# EUROPEAN PATENT APPLICATION

(11) **EP 3 811 969 A1**
(43) Date of publication of application: **28.04.2021**
(21) Application number: 20170658.7
(22) Date of filing: 21.04.2020
(51) Int. Cl.: A61K 39/00, A61K 39/385, A61P 43/00

(54) **IMMUNIZATION SCHEME FOR VARIANT SURFACE GLYCOPROTEIN CARRIERS**

(30) Priority: 24.10.2019 WO PCT/EP2019/079063
(71) Applicant: Deutsches Krebsforschungszentrum, Stiftung des öffentlichen Rechts, 69120 Heidelberg (DE)
(72) Inventor: PAPAVASILIOU, Nina, 69120 Heidelberg (DE); TRILLER, Gianna, 69120 Heidelberg (DE); STEBBINS, Erec, 69120 Heidelberg (DE); VERDI, Joseph, 69120 Heidelberg (DE)
(74) Representative: Kuttenkeuler, David

(57) **Abstract**

The invention pertains to an immunization scheme for inducing or amplifying an immune response involving Variant Surface Glycoproteins as carriers for antigenic structures against which the immune response is targeted. The invention is based on a specific priming and boosting schedule using the array presented and soluble VSG variants. Surprisingly, the immunization scheme of the invention elicits a strong and long-lasting antibody response in the immunized subject and therefore is applicable in vaccination approaches, immunotherapy and in the production of novel antibodies.

## Description

### FIELD OF THE INVENTION

The invention pertains to an immunization scheme for inducing or amplifying an immune response involving Variant Surface Glycoproteins as carriers for antigenic structures against which the immune response is targeted. The invention is based on a specific priming and boosting schedule using the array presented and soluble VSG variants. Surprisingly, the immunization scheme of the invention elicits a strong and long-lasting antibody response in the immunized subject and therefore is applicable in vaccination approaches, immunotherapy and in the production of novel antibodies.

### DESCRIPTION

Abuse of and addiction to drugs present major problems to medical, social, and economic health, both for individuals and their families, and for society as a whole. Especially in the U.S.A, abuse of prescription opioid pain medications, such as OxyContin® and Vicodin®, has reached epidemic levels and is now a public health crisis of grave concern. In other regions (e.g., Asia, Africa and the Middle East), Tramadol abuse is on a rapid rise (UNODC World Drug Report 2017). Every facet of society has been affected by the relative availability of these drugs, their perceived safety, and the over-prescription of these drugs by medical prescribers.

The path to addiction often derives from medically sanctioned administration for pain management; however, for a substantial fraction of the population (estimated at 10-15%) this can quickly lead to addiction, followed by illicit use of prescription opioids. Of particular concern is the prevalence of illicit use of these drugs by teenagers, and the rapid increase in emergency room visits (reaching 1.2 million in 2009; DAWN report, 2010) as well as in cases of fatal overdoses resulting from abuse (with deaths from overdose, estimated at 72,000 in 2017, surpassing deaths from breast cancer for the first time, with no end in sight). Currently, treatment options are limited to supervised, mediated controlled withdrawal in rehab/detox clinics (for example using methadone or buprenorphine).

Methadone maintenance, developed at the Rockefeller University for the treatment of heroin addiction, remains the most effective and widely used pharmacotherapeutic for any addictive disease (Dole et al., 1966; Kreek, 2000). But treatment often requires years and retention rates are very poor, raising interest in additional options and/or preventatives of abuse in the first place. One approach that has emerged for addiction is a vaccine to raise neutralizing antibodies against a particular drug target (Shen et al., 2012), an option which has been explored most extensively for cocaine (Fox et al, 1996; Shen and Kosten, 2011) and more recently for heroin derivatives (Pravetoni and colleagues, 2012, 2017 and 2018). The idea of immunotherapy to cure opioid use disorder (or at least to prevent overdose) is based on the notion that if a drug conjugated to a carrier protein can elicit a long-lasting immune response, in which antibodies directed to the chemical structure of the drug develop, then circulating antibodies can bind to the drug upon administration by the user, preventing the drug from getting to the site of action in the brain.

Successful vaccination is defined as the elicitation of high antibody titers and memory B cells. High titers are produced by the recruitment of antigen-specific B cell, their activation and differentiation into antibody-producing plasma blasts. Memory B cells can induce a recall (memory) response which requires, in addition, cognate T-cell help. Most successful vaccines are made of a single component in adjuvant (e.g. a viral particle), that is given as priming immunization and multiple follow-up boosts. But this does not work for vaccines against small molecules or short, well-defined epitopes in isolation. The immune system physiologically responds to large (nano to micrometer) entities (viruses, bacteria) but not to entities the size of a few nucleotides or amino acids. In the context of antibody elicitation against small molecules or defined small epitopes in general, specific B cell recruitment can only be elicited when the small epitope (also known as the hapten) is attached to a much larger protein (known as the carrier), and delivered with adjuvant (which is meant to "artificially" elicit cognate T-cell help). But because haptens are a small fraction of the size of the carrier, most of the immune response is raised against the carrier, and this includes the generation of cognate T-cell help. Consequently, whereas this approach can generate reasonable titers, it has failed in generating memory every time it has been attempted.

Thus, it is an objection of the invention to provide a vaccination and immunization approach which allows for the generation of a stable and long-lasting memory immune response in a subject.

### BRIEF DESCRIPTION OF THE INVENTION

Generally, and by way of brief description, the main aspects of the present invention can be described as follows:
In **a first aspect,** the invention pertains to a method for eliciting an immune response, and/or for amplifying a pre-existing immune response, against an antigenic compound in a subject, comprising at least one priming-immunization step and at least one boosting-immunization step, wherein the at least one boosting-immunization step is performed after the at least one priming-immunization step, and wherein: (i) the at least one priming-immunization step comprises an immunization of the subject with an array of immunization-carrier-fusions, and wherein each immunization-carrier-fusion is composed of a carrier protein fused to the antigenic compound; and (ii) the at least one boosting-immunization step comprises an immunization of the subject with the immunization-carrier-fusion used in (i) in a non- arrayed, preferably soluble form.
In **a second aspect,** the invention pertains to a method for generating antibodies against an antigenic-compound, the method comprising performing in a non-human animal the method of the first aspect, and isolating from the non-human animal newly generated antibodies, or B-cells producing antibodies, wherein the antibodies are directed against the antigenic compound.
In **a third aspect,** the invention pertains to an antibody, or an antibody producing B-cell, generated and/or isolated according to the method of the second aspect.
In **a fourth aspect,** the invention pertains to an immunization kit, the immunization kit comprising (a) an array or aggregate of immunization-carrier protein recited in the first aspect and (b) a non-aggregated and/or non-arrayed, preferably soluble form, of the immunization-carrier protein of (a); wherein the immunization-carrier protein of (a) and (b) are capable to be fused to an antigenic compound to obtain an immunization-carrier-fusion recited in the first aspect.
In **a fifth aspect,** the invention pertains to a method of treating or preventing an adverse condition in a subject by eliciting an immune response, and/or for amplifying a pre-existing immune response, against an antigenic compound which causes or is associated with the adverse condition, the method comprising performing the steps of the method of the first aspect.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

### Definitions:

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "antigenic" in context of the invention shall refer to a feature of the particle of the invention to induce a specific immune response when used as an immunogen in a vaccination or immunization procedure.

The term "variant surface glycoprotein", which is abbreviated as "VSG", refers to a family of ∼60 kDa proteins which densely pack the cell surface of protozoan parasites belonging to the genus *Trypanosoma.* The parasite has a large cellular repertoire of antigenically distinct VSGs (∼1500 complete and partial (pseudogenes)) which are expressed from a bloodstream expression site (BES, ES) in a polycistron by RNA polymerase I (recruited to a ribosomal-type promoter) with other ES-associated genes (ESAGs), of which transferrin receptor (Tfr: ESAG6, ESAG₇) is one. Only one VSG gene is expressed at a time, as only one of the ∼15 ES are active in a cell. VSG expression is 'switched' by homologous recombination induced by double-strand breaks of a silent basic copy gene from an array (directed by homology) into the active telomerically-located expression site. VSG annotation, protein sequences and gene sequences are derivable from public databases such as www.ensemble.org. A collection of VSGs of *Trypanosoma brucei brucei* (Lister 427 strain) is published in Cross GA. et al., Mol. Biochem. Parasitol. 2014 Jun; 195(1):59-73. Doi: 10.1016/j.molbiopara.2014.06.004. Preferred VSGs in context of the present invention are *T. brucei* VSGs, more preferably VSG1, VSG2, VSG3 or ILTat1.24. A VSG according to the invention is preferred which is characterized by an N-terminus which is located 3-dimensionally within the VSG at a position which is, when the VSG is present within a VSG coat, for example on a trypanosome cell, located sufficiently close to the accessible outer coat-surface, that the addition of a linker sequence (preferably not more than 100 amino acids, preferably not more than 50 amino acids, and most preferably not more than 20 amino acids in length) allows for modification of the so extended or not extended N-terminus of the VSG protein. Preferably any sequence that is N-terminal inserted into a VSG sequence will be inserted right before the starting methionine (M). In further embodiments the insertion is immediately downstream of the signal peptide cleavage site. Preferred VSG sequences to be used in context of the invention are the VSGs comprising an amino acid sequence according to any one shown in SEQ ID NO: 1 to SEQ ID NO: 5.

A "VSG type" or "type of VSG" shall pertain to a family member of the VSG family, preferably one of trypanosomal VSGs.

The term "engineered VSG" or "eVSG" shall refer to any VSG protein comprising an artificial modification compared to the wild-type sequence of said VSG. Preferably the eVSG is a non-naturally-occurring sequence. The VSG can be modified to become an eVSG either by post-translational modification, chemical modification, genetic engineering of the VSG coding sequence and any other means known to the skilled person for protein modification.

The terms "immunogenic compound" or alternatively "immunogen" as used herein, encompasses any kind of compound, or structure, capable of eliciting an immune response in a host. Preferably, but not necessarily, an immunogenic compound according to the invention comprises an amino acid sequence or is a small molecular compound. A small molecule in context of the herein described invention shall be any compound having a molecular mass significantly lower than for example complex macromolecules or proteins, so preferably a small molecule has a molecular weight of less than 50 kDa, preferably less than 20 or 10 kDa.

The term "immunization-carrier-fusion" in context of the present invention is a fusion of a carrier protein suitable for immunization purposes, fused to, either covalently, which is preferred, or non-covalently, to an immunogen or antigenic compound for which antibodies or immune responses are to be generated in accordance with the invention. A carrier protein is preferably a VSG or a variant thereof as described herein. The immunogen or antigen is also one as described herein but preferably is a small molecule, such as a small molecule drug.

An "array" or "aggregate" of a carrier protein or immunization-carrier-fusion in accordance with the herein disclosed invention pertains to an ordered arrangement of multiple such proteins in close proximity to each other, for example as in a VSG coat on a trypanosome. While the array is preferably provided as a protein carrier coat on a biological cell, other particles known to the skilled artisan can also be used to perform the immunization of the invention, as long as the particle provides multiple immunization-carrier-fusions of the invention.

Contrary thereto, the term "soluble immunization-carrier-fusion" pertains to solubilized single immunization-carrier-fusion proteins.

The term "sortase," as used herein, refers to a protein having sortase activity, i.e., an enzyme able to carry out a transpeptidation reaction conjugating the C-terminus of a protein to the N-terminus of a protein via transamidation. The term includes full-length sortase proteins, e.g., full-length naturally-occurring sortase proteins, fragments of such sortase proteins that have sortase activity, modified (e.g., mutated) variants or derivatives of such sortase proteins or fragments thereof, as well as proteins that are not derived from a naturally occurring sortase protein, but exhibit sortase activity. Those of skill in the art will readily be able to determine whether or not a given protein or protein fragment exhibits sortase activity, e.g., by contacting the protein or protein fragment in question with a suitable sortase substrate under conditions allowing transpeptidation and determining whether the respective transpeptidation reaction product is formed.

Suitable sortases will be apparent to those of skill in the art and include, but are not limited to, sortase A, sortase B, sortase C, and sortase D type sortases. Suitable sortases are described, for example, in Dramsi S, Trieu-Cuot P, Bierne H, Sorting sortases: a nomenclature proposal for the various sortases of Gram-positive bacteria. Res. Microbiol. 156(3):289-97, 2005; Comfort D, Clubb RT. A comparative genome analysis identifies distinct sorting pathways in gram-positive bacteria. Infect Immun., 72(5):2710-22, 2004; Chen I, Dorr B M, and Liu D R., A general strategy for the evolution of bond-forming enzymes using yeast display. Proc Natl Acad. Sci. USA. 2011 Jul. 12; 108(28):11399; and Pallen, M. J.; Lam, A. C.; Antonio, M.; Dunbar, K. TRENDS in Microbiology, 2001, 9(3), 97-101; the entire contents of each of which are incorporated herein by reference). Any known sortase can be used as a starting enzyme in an evolution strategy provided herein, and the invention is not limited in this respect. For example, the present invention encompasses embodiments relating to a sortase A from any bacterial species or strain. Those of skill in the art will appreciate that any sortase and any sortase recognition motif can be used in some embodiments of this invention, including, but not limited to, the sortases and sortase recognition motifs described in Ploegh et al., International PCT Patent Application, PCT/US2010/000274, filed Feb. 1, 2010, published as WO 2010/087994 on Aug. 5, 2010; Ploegh et al., International Patent Application PCT/US2011/033303, filed Apr. 20, 2011, published as WO 2011/133704 on Oct. 27, 2011; Liu et al., U.S. provisional Patent Application 61/662,606, filed on Jun. 21, 2012; and Liu et al., U.S. provisional Patent Application 61/880,515, filed on Sep. 20, 2013; the entire contents of each of which are incorporated herein by reference. The invention is not limited in this respect.

The term "sortase substrate," as used herein refers to a molecule or entity that can be utilized in a sortase-mediated transpeptidation reaction. Typically, a sortase utilizes two substrates-a substrate comprising a C-terminal sortase recognition motif, and a second substrate comprising an N-terminal sortase recognition motif and the transpeptidation reaction results in a conjugation of both substrates via a covalent bond. In context of the invention the "C-terminal sortase recognition motif' is also referred to as "sortagging donor sequence", whereas the term "N-terminal sortase recognition motif' is referred to as "sortagging acceptor sequence". In preferred embodiments, the C-terminal and N-terminal recognition motifs are comprised in different amino acid sequences, for example, one N-terminally of the VSG, and the other linked to the immunogen such that there is a free carboxyl group at the end of the sortagging donor site. Some sortase recognition motifs are described herein and additional suitable sortase recognition motifs are well known to those of skill in the art. For example, sortase A of *S. aureus* recognizes and utilizes a C-terminal LPXT(G/A) motif, preferably LPXTG, (where X is any amino acid and glycine cannot be a free carboxylate) and an N-terminal oligoglycine (G₁₋₅) - the index indicating the number of guanine compounds - , preferably G₃ or G₅, motif in transpeptidation reactions. Additional sortase recognition motifs will be apparent to those of skill in the art, and the invention is not limited in this respect. A sortase substrate may comprise additional moieties or entities apart from the peptidic sortase recognition motif. For example, a sortase substrate may comprise an LPXTG/A motif, the N-terminus of which is conjugated to any agent, (e.g. a peptide or protein, a small molecule, a binding agent, a lipid, a carbohydrate, or a detectable label). Similarly, a sortase substrate may comprise an oligoglycine (G₁₋₅) motif, preferably G₃ or G₅, the C-terminus of which is conjugated to any agent, e.g., a peptide or protein, a small molecule, a binding agent, a lipid, a carbohydrate, or a detectable label. Accordingly, sortase substrates are not limited to proteins or peptides but include any moiety or entity conjugated to a sortase recognition motif.

The terms "sortagging" in context of referring to a donor or acceptor sequence is sometimes also referred to as "sortase donor sequence", or "sortase acceptor sequence".

A "pre-ieVSG" in accordance with the invention is an eVSG having a free N-terminal acceptor site for the addition of an immunogenic compound. For example the pre-ieVSG is expressed on the coat of a trypanosome cell and comprises a free sortase acceptor sequence outside (such as accessible on the surface of) the VSG coat. Using a sortase enzyme in a sortase reaction, the pre-ieVSG can be fused to an immunogenic compound which is coupled to a sortase donor sequence.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption-delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The term "priming" or "priming immunization" or "priming immunization step" relates to a method step of "priming" immunization, comprising the initial administration of one or more antigens to a subject in preparation for subsequent administration(s) of the same antigen. Specifically, the term "priming", as used herein, defines a first immunization using an antigen which induces an immune response to the desired antigen and recalls a higher level of immune response to the desired antigen upon subsequent re-immunization with the same antigen when administered in the context of the same or a different vaccine or immunogen delivery system. Specifically as used in this application, a "priming immunization" refers to the administration of a composition comprising a preparation containing an array of VSG carrier proteins coupled to the antigen, preferably the array is a VSG coat. As used herein, a "priming immunogenic composition or preparation" refers to a preparation containing such VSG carrier antigen fusions of the invention.

Another component of the methods and compositions of the present invention is the use of a "boosting immunization", or a "boost", or "boosting-immunization step" which means the administration of a composition delivering the antigen or antigenic compound as encoded in the priming immunization, but utilizing a preparation with a different form of the carrier platform, and preferably is not an array and/or not an aggregate of the VSGs carrier protein antigen fusions described herein, and most preferably is a soluble VSG carrier antigen fusion of the invention, and most preferably of the same VSG; a whole organism or extract and combinations thereof. A boost is sometimes referred to as an anamnestic response, i.e. an immune response in a previously sensitized subject. A boosting immunization or a boosting immunogenic composition can comprise multiple doses, which may be the same or different amounts.

The term "small molecule" or "small molecular compound" or similar expressions is intended to include organic molecules with a molecular weight of 100 Dalton to 10,000 Dalton. Preferably, the small molecule has a molecular weight of 200 to 5,000 Dalton. In particular, the small molecule may be a small chemical fragment. The term "small molecule" is not intended to include fluorescent dyes. Further, the term "small molecule" is not intended to include nucleic acid polymers, large proteins and polysaccharides. However, the building blocks of nucleic acid polymers, large proteins and polysaccharides, i.e. nucleotides, amino acids and monosaccharides, and small oligomers thereof can be considered as small molecules. In particular, the term "small molecule" is intended to include peptides with a size of 250 to 8,000 Dalton.

By "vaccine," is meant a composition comprising an immunogen or antigenic compound whichh, when inoculated into a subject, has the effect of stimulating a cellular immune response comprising a T-cell response and/or a humoral immune response comprising a B-cell response generally resulting in antibody production. The T cell response can be a cytotoxic T-cell response directed against an organism that expresses the antigen. However, the induction of a T-cell response comprising other types of T cells by an immunogen disclosed herein is also contemplated. A B-cell response results in the production of antibody that binds to the antigen. The vaccine can serve to elicit an immune response in the mammal which serves to protect the mammal against a disease. The terms "vaccine," "immunogen," "immunogenic polypeptide,", "antigen", "antigenic compound" and the like may be used interchangeably. The term "vaccine" does not in any way connote that the composition is capable of fully preventing a disease in a vaccinated subject, or providing any specific or general level of protection against an infectious agent. In certain aspects a "vaccine" can be ineffective in certain subjects while inducing an immune response in other subjects.

In the **first aspect,** the invention pertains to a method for eliciting an immune response, and/or for amplifying a pre-existing immune response, against an antigenic compound in subject, comprising at least one priming-immunization step and at least one boosting-immunization step, wherein the at least one boosting-immunization step is performed after the at least one priming-immunization step, and wherein: (i) the at least one priming-immunization step comprises an immunization of the subject with an array of immunization-carrier-fusions, and wherein each immunization-carrier-fusion is composed of a carrier protein fused to the antigenic compound; and (ii) the at least one boosting-immunization step comprises an immunization of the subject with the immunization-carrier-fusion used in (i) in a non-aggregated and/or non-arrayed, preferably soluble form.

In the present invention it was surprisingly discovered that immunization procedures involving VSG proteins as carrier proteins for smaller immunogens are highly efficient when during the priming step an array or dense VSG-immunogen fusions are administered, and subsequently, the same VSG-immunogen construct is used in soluble form for one or more boosting steps.

The method of claim 1, wherein the immunization-carrier-fusion is an antigenic particle coated with an engineered variant surface glycoprotein (eVSG), wherein the eVSG comprises the antigenic compound covalently linked, optionally via a linker, to the N-terminus of the VSG carrier protein.

A preferred embodiment of the antigenic particle of the invention includes that the immunogenic compound is covalently linked, optionally via a linker, to the N-terminus of the VSG. The immunogenic compound may be linked to the VSG by any means known to the skilled artisan, including any chemical reaction, preferably click-chemistry, cross-linking, or use of biological entities such as enzymes, ligases, protein-protein interactions and the like. The term "click-chemistry" in context of the invention shall refer to chemistry tailored to generate covalent bonds quickly and reliably by joining small units comprising reactive groups together (see H. C. Kolb, M. G. Finn and K. B. Sharpless, 2001). Any variants of such approaches may be used to connect the immunogenic compound to the VSG in accordance with the invention.

In general, the linking of the VSG to the immunogenic compound may include the use of any linking means or linker, which in context of the herein disclosed invention refers to the means by which the VSG and the immunogenic compound are linked or connected to form an eVSG. The one or more linkers or linking means for linking the VSG and the immunogenic compound may be any structurally suitable means to connect the two. Exemplary linkers include the use of one or more amino acids which may be used to form a peptide, in some embodiments having a modified peptide backbone, a small chemical scaffold, a biotin-streptavidin, an organic or inorganic nanoparticle, a polynucleotide sequence, peptide-nucleic acids, an organic polymer, or an immunoglobulin Fc domain. The means for linking can comprise covalent and/or noncovalent bonds. The one or more linkers can include various sequences or other structural features that provide various functions or properties. For example, the one or more linkers can contain structural elements to allow the eVSG to be derivatized.

In one preferred embodiment of the antigenic particle of the invention, the antigenic particle comprises the linker, which is an N-terminal extension of the wild-type VSG N-terminal sequence, and preferably comprises 5 to 30 amino acids, preferably 10 to 20, more preferably about 15 amino acids. The linker here is used in order to render the N-terminus of the VSG more accessible to modifications, for example using enzymes, when the VSG is provided in context of an assembled VSG coat. The linker sequence is preferably introduced into the VSG by genetic engineering the VSG coding gene. Preferably the linker is introduced immediately C-terminal to, if present, a signal peptide sequence which allows for a cell surface targeting of the VSG protein. Signal peptides are usually cleaved after export. Hence, the term "signal peptide" as used herein refers to amino-terminal amino acid residues that, when attached to a target polypeptide, permits the export of the target polypeptide from the cell and cleavage of the signal peptide. A preferred linker in accordance with the invention is a G4S linker, however, any other protein linker which essentially does not interfere with VSG folding can be used in context of the invention. A G4S linker in accordance with the herein disclosed invention may comprise a multiplicity of consecutive 4GS, such as a preferred linker is a G4S linker which may have the amino acid sequence of GGGGSGGGGSGGGGS (SEQ ID NO: 8).

In an additional or alternative embodiment of the invention the VSG may include a sortase acceptor sequence of any sorts. Such a sequence is preferably attached to the N-terminus of the VSG sequence in such a way that it is coat-surface accessible by a sortase enzyme. If the eVSG sequence comprises an N-terminal linker, the sortase acceptor sequence is located at the N-terminus of the linker. Exemplary sortase acceptor sequences are di-Alanine (AA-) or di-Glycine (GG-), or any other sequence that can be used for sortase-mediated ligation.

Hence, in some embodiments of the invention the immunogenic compound is linked to the VSG preferably by using a sortase enzyme.

In particular preferred embodiments of the invention, thus, the antigenic compound forms a linkage to the carrier protein via a sortagging donor sequence, such as (G)3SLPSTGG and a sortagging acceptor sequence by covalent connection mediated by a sortase. It may be preferred that the linkage composed of the sortagging donor sequence and the sortagging acceptor sequence comprises the sequence -(G)3SLPSTGAA-, or a sortagging-functional variant thereof.

Hence, in preferred embodiments, the immunization-carrier-fusion has the following (covalent) structure from N- to C-terminus: antigenic compound, a sortagging donor sequence, a sortagging acceptor sequence, a linker, a VSG protein sequence.

The immunization and vaccination protocol of the invention is particularly in application where the antigenic compound is a small molecule drug (of less than 10kD), such as therapeutic compound, a sugar, an allergen, and/or a dependence causing substance. Even more preferably, the antigenic compound is a dependency causing substance selected from (i) delta-9-tetrahydrocannabinol (THC) or Synthetic cannabinoids, such as classical cannabinoids, non-classical cannabinoids, hybrid cannabinoids, aminoalkylindoles, and eicosanoids; for example Δ9-THC HU-210, (C8) CP 47,497, JWH-018, AM-2201 (Fluorinated JWH-018), UR-144, XLR-11 (Fluorinated UR-144), APICA, STS-135 (Fluorinated APICA). AB-PINACA, PB-22, 5F-PB-22 (Fluorinated PB-22); or (ii) methamphetamine and derivatives thereof such as 3,4-methylenedioxy-methamphetamine (MDMA)Ecstasy/Molly; or (iii) a synthetic cathinone like alpha-pyrrolidinopentiophenone (alpha-PVP); or (iv) an opioid including heroin, synthetic opioids such as fentanyl or related compounds such as carfentanyl, and other opioid pain relievers, such as oxycodone (OxyContin®), hydrocodone (Vicodin®), codeine, morphine, desomorphine (Krokodil); or (v) steroids (anabolic substances), or is nicotine.

Opium alkaloids and derivatives in accordance with the invention are selected from opium alkaloids: phenanthrenes like codeine; morphine; thebaine; oripavine or mixed opium alkaloids, including papaveretum; esters of morphine like diacetylmorphine (morphine diacetate; heroin); nicomorphine (morphine dinicotinate); dipropanoylmorphine (morphine dipropionate); diacetyldihydromorphine; acetylpropionylmorphine; dmaDesomorphine; methyldesorphine; dibenzoylmorphine; ethers of morphine like dihydrocodeine; ethylmorphine; heterocodeine

Also included are semi-synthetic alkaloid derivatives such as buprenorphine; etorphine; hydrocodone; hydromorphone; oxycodone; oxymorphone.

Also included are synthetic opioids such as anilidopiperidines like fentanyl; alphamethylfentanyl; alfentanil; sufentanil; remifentanil; carfentanyl; ohmefentanyl; also phenylpiperidines like pethidine (meperidine); ketobemidone; MPPP; allylprodine; prodine; PEPAP; promedol.

Diphenylpropylamine derivatives that are included comprise propoxyphene; dextropropoxyphene; dextromoramide; bezitramide; piritramide; methadone; dipipanone; levomethadyl acetate (LAAM); difenoxin; diphenoxylate; loperamide.

Further included are: Benzomorphan derivatives like Dezocine, Pentazocine, Phenazocine; Oripavine derivatives like Buprenorphine, Dihydroetorphine, Etorphine; Morphinan derivatives like Butorphanol; Nalbuphine; Levorphanol; Levomethorphan; Racemethorphan; Others like Lefetamine; Menthol (Kappa-Opioid agonist); Meptazinol; Mitragynine; Tilidine; Tramadol; Tapentadol; Eluxadoline; AP-237; 7-Hydroxymitragynine.

In some embodiments, the immunogenic compound used in context of the invention is an allergen, or any allergy inducing fragment thereof. An "allergen" in context of the herein disclosed invention means any compound capable of eliciting allergy as defined below. An allergen is usually a biopolymer, mostly a peptide or protein, or biological complex sugar molecules, or any fragments or monomers thereof. It is particular preferred to use the present invention for producing antibodies against allergens in host animals. Such antibodies may then be used as a therapeutic against the allergy in a, for example, human subject suffering from the allergy induced by the respective allergen.

The term "allergy" means any type of hypersensitivity reaction to an environmental allergen mediated by immunological mechanisms, including Type I-IV hypersensitivity reactions, including allergic rhinitis, asthma and atopic dermatitis.

In another embodiment the immunogenic compound to be used in context of the invention can be a nucleic acid or a nucleobase derivative or variant, such as variants ogf RNA or DNA nucleobases for which antibodies are needed.

In some additional or alternative embodiments of the invention the antigenic particle comprises a particle which is a biological cell, a vesicle, a nanoparticle or a bead. Preferably the particle is selected to be capable of being coated by VSG proteins. Most preferably the particle is a biological cell, for example of a micro-organism, preferably a protozoan organism, more preferably a trypanosome, more preferably *T. brucei.* In some embodiments the trypanosome is an enzyme glycophosphatidylinositol phospholipase C (GPI-PLC)-negative trypanosome. Using a GPI-PLC-negative *T. brucei* strain has the advantage that after inactivation of the cell, the VSG coat is not disassembled, but remains intact. Thus, in other additional or alternative embodiments the biological cell is a non-living, preferably non-infective biological cell, such as an inactivated biological cell, preferably a UV-crosslinked cell.

In some embodiments the antigenic particle of the invention may comprise two or more different eVSGs on its surface. Thereby the antigenic particle may be used to display two or more different (or 3, 4, 5, 6 etc.) immunogenic compounds on its surface coat. For example, the different immunogenic compounds may comprise different immunogenic epitopes of one antigen or may comprise different epitopes of different antigens.

In some other embodiments of the invention the eVSG comprises the immunogen as one or more immunogenic amino acid sequence(s) (that preferably is (are) genetically) inserted into the VSG sequence. Preferably the inserted immunogenic sequence is a xenogenic sequence, and most preferably is inserted into the VSG sequence without causing a deletion in the wild-type VSG sequence. In some embodiments the insertion is located in a surface loop of VSG, such as a region between two secondary structural motifs in the VSG, and preferably wherein said surface loop is located in a 3-dimensional position within the VSG which is surface presented when the VSG is comprised or assembled in a VSG coat, preferably which is surface-accessible and can be presented to the immune system.

In some embodiments of the invention the immunogenic (antigenic) compound comprised in the antigenic particle is a disease-associated antigen, such as a peptide antigen, and the disease is preferably selected from a proliferative disorder, an infectious disease, an inflammatory disorder, an immune deficiency disorder or an autoimmune disorder; or the disease is a non-communicable disease. Most preferably the immunogenic compound is an antigen associated with an infectious disease or proliferative disorder; preferably the antigen is associated with the disease.

A preferred antigenic particle in context of the invention is a living or an inactivated trypanosome cell having an intact VSG coat, wherein the VSG coat comprises one or more immunogenic engineered VSG (ieVSG), preferably a high percentage of an ieVSG (20% or higher, 40% or higher, 50% or higher, 60% or higher, 70% or higher, 80% or higher, with increasing preference), wherein the ieVSG comprises an eVSG protein N-terminally linked to an immunogenic compound. Preferably the linkage is provided by a sortase-mediated reaction.

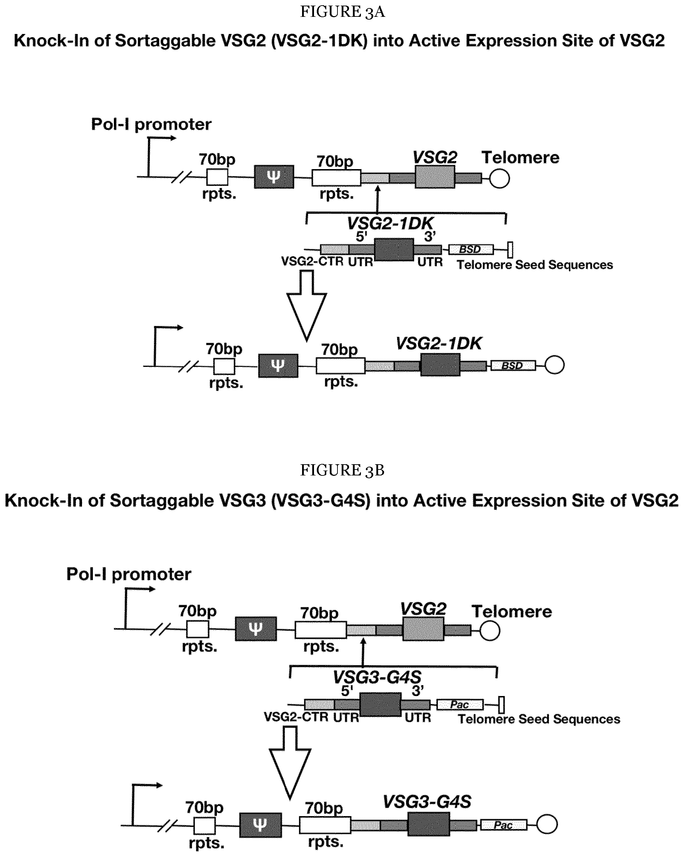

Further provided are nucleic acid constructs comprising an eVSG of the invention suitable for the introduction into the VSG expression locus of trypanosomes. Preferably the nucleic acid construct for introduction into a trypanosome has a structure as shown in any one of Figures 3A to 3C. Generally, a nucleic acid of the invention comprises the following elements in this order: a co-transposed region (CTR) of a VSG, for example about 1156 bp preceding the VSG2 open reading frame in Bloodstream Expression Site -1 (BES1) of the *T. brucei brucei* (Lister 427 strain) genome, immediately followed by the coding region for the eVSG in accordance with the invention, and a telomere seed sequence, for example a telomere seed sequence of about 150 to 250, preferably 200 bp. The construct may further comprise a resistance gene, for example blasticidin, puromycin or hygromycin, preferably following the eVSG coding region. Methods for integrating such constructs into the trypanosome genome are shown in Pinger et al., 2017 (Nature Communications), of which the materials and methods on pages 7 and 8 are incorporated herein by reference.

In other preferred embodiments the antigenic compound is a biological antigen, for example, a peptide or nucleic acid immunogen, wherein the biological antigen is derived from a disease associated antigen structure or molecule. Disease associated antigens may be any antigen that specifically and selectively is expressed in cells or tissue associated with the disease, or in any pathogen causing or being associated with the disease. Such biological antigens may be associated to a cancer or tumour, or may be associated to an autoimmune or inflammatory disease. Furthermore, biological antigens may be associated with a bacterial, fungal or viral disease, or any other unicellular pathogenic organisms, or a pathogenic or parasitic animal. Typically, such biological antigens are peptide or nucleic acid molecules having a specific sequence only found in the pathogen or disease associated cell or tissue. Preferred peptides are small peptide fragments of a protein expressed as a component of a virus, such as and preferably of a corona virus (such as COVID-19).

Preferred VSG proteins for use in context of the herein disclosed invention are VSG derived from *Trypanosoma brucei.*

In context of the herein disclosed invention, the array or aggregate of immunization-carrier-fusions constitutes a particle coated with multiple immunization-particle fusions. Hence, such immunization-particle fusions are displayed in an array on the surface of the particle. Preferably an array or aggregate is present if there are more than one immunization-carrier-fusion per particle, preferably more than two, more preferably more than three, or more than 4, 5, 8, 10, 15, 20, more preferably more than 50 such molecules per particle. The density of the immunization-carrier-fusion on the particle will be dependent on the particle used. Higher densities may be obtained by using larger particles, such as an inactivated trypanosome, or a trypanosome coat.

A particle that can be used in context of the present invention may be not only objects such as microscopic beads (e.g., chromatographic and fluorescent beads), latex, glass, silica or paramagnetic beads, but also includes other porous and/or biomaterials such as liposomes, vesicles and other emulsions. Beads ranging in size from 0.1 micron to 1 mm can be used in the invention and are therefore encompassed with the term "particle" as used herein. The term particle also encompasses biological cells, as well as beads and other microscopic objects of similar size (e.g., from about 0.1 to 120 microns, and typically from about 1 to 50 microns) or smaller (e.g., from about 0.1 to 150 nm). Preferably a particle for use in context of the herein disclosed invention is selected from the group consisting of a liposome, micelle, a bead, a vesicle and a cell. In some preferred embodiments, the cell is a trypanosome cell, preferably an inactivated, such as UV inactivated, trypanosome cell (VSG-coat), or any fragments thereof. Even more preferably the cell is an enzyme phospholipase C (or PLC) negative trypanosome.

In preferred embodiments of the present invention, the immunization-carrier used in the method is a VSG protein, and wherein the VSG protein used as carrier in (i) and (ii) are derived from the same VSG protein type, for example the VSG in both (i) and (ii) are VSG3 of *Trypanosoma brucei.* The "same" VSG protein in this context shall refer to a mostly identical parent VSG protein, meaning VSG family member, such as the protein sequence used between the carrier used in (i) compared to the carrier used in (ii) shares higher sequences identity to each other as either of them to any given third VSG protein family member. The members of VSG proteins is disclosed for example in Cross GAM, Kim HS, Wickstead B. Capturing the variant surface glycoprotein repertoire (the VSGnome) of Trypanosoma brucei Lister 427. Mol Biochem Parasitol. 2014; 195:59-73 or on "http://tryps.rockefeller.edu".

In some embodiments that are preferred, the present immunization method comprises at least two priming-immunization steps, and wherein all of the priming immunization steps are performed prior to any of the boosting-immunization steps. In some embodiments there might be three or four priming-immunization steps before any boosting-immunization step is performed.

In particular embodiments it is preferred that at least two boosting-immunization steps are preformed, and that all of the boosting immunization steps are performed after (or subsequent to) any of the priming-immunization steps.

More preferred embodiments of the invention relate to the method wherein at least two priming-immunization steps and at least two boosting-immunization steps are performed. Again, any of the priming-immunization steps are performed prior to any of the boosting-immunization steps. Some preferred embodiments may include any combination of repeated priming and boosting immunization step.

A characteristic of the herein disclosed invention is that the method of the invention elicits a long-lasting, such as more than 1 preferably more than 2 months, antibody response (with antibody titers preferably higher than 100 µg/mL) in the subject that is immunized. Hence, the method is preferably a method for eliciting a long-lasting, such as more than 1 preferably more than 2 months, antibody response (with antibody titers preferably higher than 100µg/mL) in the subject that is immunized.

A subject according to the invention is in particular embodiments selected from an animal having an adaptive immune system, such as vertebrates such as birds (chicken), and mammals such as mouse, rabbit, camel, goat, rat, dog, cat, monkey, hamster or other mammals, or is a human, such as a human patient in need of an elicited or amplified immune response.

In context of the invention the immunogenic compound is a small molecule, a nucleic acid, or a peptide. In other embodiments the immunogenic compound is considered as a small molecule, a nucleic acid, a carbohydrate, a lipid or a peptide, or any combination of these or other chemical entities).

A preferred embodiment of the antigenic particle of the invention includes that the immunogenic compound is covalently linked, optionally via a linker, to the N-terminus of the VSG. The immunogenic compound may be linked to the VSG by any means known to the skilled artisan, including any chemical reaction, preferably click-chemistry, cross-linking, or use of biological entities such as enzymes, ligases, protein-protein interactions and the like. The term "click-chemistry" in context of the invention shall refer to chemistry tailored to generate covalent bonds quickly and reliably by joining small units comprising reactive groups together (see H. C. Kolb, M. G. Finn and K. B. Sharpless, 2001). Any variants of such approaches may be used to connect the immunogenic compound to the VSG in accordance with the invention.

In general, the linking of the VSG to the immunogenic compound may include the use of any linking means or linker, which in context of the herein disclosed invention refers to the means by which the VSG and the immunogenic compound are linked or connected to form an eVSG. The one or more linkers or linking means for linking the VSG and the immunogenic compound may be any structurally suitable means to connect the two. Exemplary linkers include the use of one or more amino acids which may be used to form a peptide, in some embodiments having a modified peptide backbone, a small chemical scaffold, a biotin-streptavidin, an organic or inorganic nanoparticle, a polynucleotide sequence, peptide-nucleic acids, an organic polymer, or an immunoglobulin Fc domain. The means for linking can comprise covalent and/or noncovalent bonds. The one or more linkers can include various sequences or other structural features that provide various functions or properties. For example, the one or more linkers can contain structural elements to allow the eVSG to be derivatized.

In **a second aspect,** the invention pertains to a method for generating antibodies against an antigenic-compound, the method comprising performing in a non-human animal the method of the first aspect, and optionally in any single or combination of the described embodiments and variations of the method of the first aspect, and isolating from the subject (such as a non-human animal) newly generated antibodies, or B-cells producing antibodies, wherein the antibodies are directed against the antigenic compound.

In **a third aspect,** the invention pertains to an antibody, or an antibody producing B-cell, generated and/or isolated according to the method of the second aspect.

The method of the invention in the second and third aspect provides the vaccination procedure described herein for the production of antibodies against a target antigenic compound. It was surprisingly discovered that immunization according to the herein described methods yields stable antibody responses. Hence, the method of the invention is particular useful for generating new antibodies against antigens that otherwise insufficiently immunogenic.

The method of the second aspect preferably comprises a step of producing a hybridoma cell with the isolated B-cell ,and/or, a step of isolating paired heavy and light chain antibody genes and recombinantly producing them in mammalian cells.

As used herein, the term "antibody" may be understood in the broadest sense as any immunoglobulin (Ig) that enables binding to its epitope. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chains of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (Ciq) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex. VSIR is an immunoglobulin-like protein, and as such it is not (nor its variants) considered - for the purposes of the present invention - an antibody that binds to the antigenic compound of the invention.

Accordingly, in certain embodiments of the invention an antibody produced according to the herein disclosed methods can comprise an antibody heavy chain, or an antigen binding fragment thereof, and/or an antibody light chain, or an antigen binding fragment thereof.

In an alternative, and preferred, embodiment of all ABPs of the invention, the ABP is an antibody or an antigen binding fragment thereof, and the antibody is a monoclonal antibody, or wherein the antigen binding fragment is a fragment of a monoclonal antibody.

Antibodies produced according to the invention are polyclonal or monoclonal antibodies.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

Monoclonal antibodies in accordance with the present invention may be prepared by methods well known to those skilled in the art. For example, mice, rats or rabbits may be immunized in accordance with the method of the first aspect. In addition, splenocytes of such immunized animals are harvested as a pool from the animals that are administered several immunisations at certain intervals with test bleeds performed to assess for serum antibody titers. Splenocytes are prepared that are either used immediately in fusion experiments or stored in liquid nitrogen for use in future fusions or cell sorting experiments followed by antibody cloning and expression of humanised monoclonal antibodies (Tiller et al. J. Immunol. Methods 2008, 329(1-2): 112-124). Fusion experiments are then performed according to the procedure of Stewart & Fuller, J. Immunol. Methods 1989, 123:45-53. Supernatants from wells with growing hybrids are screened by e.g. enzyme-linked immunosorbent assay (ELISA) for mAb secretors. ELISA-positive cultures are cloned either by limiting dilutions or fluorescence-activated cell sorting, typically resulting in hybridomas established from single colonies. The ability of an antibody, including an antibody fragment or sub-fragment, to bind to a specific antigen can be determined by binding assays known in the art, for example, using the antigen of interest as the binding partner.

The antibodies described herein may alternatively be prepared through the utilization of the XenoMouse® technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3,1996 and International Patent Application Nos. WO 98/24893, published June 11, 1998 and WO 00/76310, published December 21, 2000. See also Mendez et al., Nature Genetics, 15:146-156 (1997). Through the use of such technology, fully human monoclonal antibodies to a variety of antigens have been produced. Essentially, XenoMouse® lines of mice are immunized according to the VSG technology of the invention, lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: e.g., Medarex - HuMab mouse, Kymab - Kymouse, Regeneron - Velocimmune mouse, Kirin - TC mouse, Trianni - Trianni mouse, OmniAb - OmniMouse, Harbour Antibodies - H2L2 mouse, Merus - MeMo mouse. Also available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (e.g. human) immunoglobulin constant region may be modified (e.g. by mutations or glycoengineering) to optimise one or more properties of such region and/or to improve the function of the (e.g. therapeutic) antibody, such as to increase or reduce Fc effector functions or to increase serum half-life. Exemplary such Fc modification (for example, Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, an antibody produced according to the invention comprises an antigen binding domain of an antibody wherein the antigen binding domain is of a human antibody. Preferably, antibody produced according to the invention comprises an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, lgG1, lgG2, lgG3, and lgG4. Human IgM subtypes include IgM, and lgM2. Human IgA subtypes include IgAi and lgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins.

In some embodiments, the antibody of the invention is an IgG antibody or fragment thereof. In some embodiments, the antibody of the invention is an IgE antibody or fragment thereof. In some embodiments, the antibody of the invention is an IgD antibody or fragment thereof. In some embodiments, the antibody of the invention is an IgA antibody or fragment thereof. In some embodiments, the antibody of the invention is an IgM antibody or fragment thereof. Preferably the antibody of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the antibody of the invention is, comprises or is derived from a rat-derived IgG, then preferably, the antibody is, comprises or is derived from, a rat IgG2a or IgG2b immunoglobulin. When the antibody of the invention is, comprises or is derived from a human-derived IgG, then more preferably, the antibody of the invention is, comprises or is derived from a human IgGi, IgG2 or IgG4, most preferably, the antibody of the invention is, comprises or is derived from a human IgG1 or IgG2

Accordingly, in particular embodiments of the invention, an antibody is preferably an IgG, IgE, IgD, IgA, or IgM immunoglobulin; preferably an IgG immunoglobulin.

An antibody of the invention is preferred, where comprising at least a portion of an immunoglobulin constant region (typically that of a human immunoglobulin) may have such (e.g. human) immunoglobulin constant region modified - for example e.g. by glycoengineering or mutations - to optimise one or more properties of such region and/or to improve the function of the (e.g. therapeutic) antibody, such as to increase or reduce Fc effector functions or to increase serum half-life.

In **a fourth aspect,** the invention pertains to an immunization kit, the immunization kit comprising (a) an array or aggregate of immunization-carrier protein recited in the first aspect and (b) a non-aggregated and/or non-arrayed, preferably soluble form, of the immunization-carrier protein of (a); wherein the immunization-carrier protein of (a) and (b) are capable to be fused to an antigenic compound to obtain an immunization-carrier-fusion recited in the first aspect.

In **a fifth aspect,** the invention pertains to a method of treating or preventing an adverse condition in a subject by eliciting an immune response, and/or for amplifying a pre-existing immune response, against an antigenic compound which causes or is associated with the adverse condition, the method comprising performing the steps of the method of the first aspect.

In context of the present invention, the immunization procedure of the invention is used in context of a vaccination or other medical treatment of an individual. Hence, in that context a further aspect of the present invention then pertains to a pharmaceutical composition comprising an antigenic particle according to the invention together with a pharmaceutically acceptable carrier and/or excipient. A pharmaceutical composition is manufactured for administration to a subject for therapy, prevention or management of a disease or disorder.

A pharmaceutical composition is preferably formulated as a vaccine composition, hence, a composition suitable for vaccination a subject in need of such a treatment.

By way of example, the pharmaceutical composition of the invention may comprise between 0.1% and 100% (w/w) active ingredient, such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%. An active ingredient of the composition of the invention is preferably an antigenic particle of the invention.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption-delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intraarterial, intravenous, intradermal, subcutaneous, oral, intraperitoneal, transdermal (topical) and transmucosal administration.

Solutions or suspensions used for parenteral, intradermal, or subcutaneous application, as well as comprising a compound of (or for use with) the invention (e.g. an antigenic particle of the invention), can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water-soluble) or dispersions and sterile powders for the extemporaneous preparation of sterile injectable solutions or dispersion. For intravenous administration, suitable carriers include physiological saline, bacteriostatic water, Kolliphor® EL (formerly Cre-mophor EL™; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyeth-ylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminium monostearate and gelatine.

Oral compositions, as well as comprising a compound of (or for use with) the invention (e.g. an antigenic particle of the invention), generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystal-line cellulose, gum tragacanth or gelatine; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavouring agent such as peppermint, methyl salicylate, or orange flavouring.

Furthermore, the compounds of (or for use with) the invention (e.g. the antigenic particle of the invention) can be administrated rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatine, hydrogenated vegetable oils, mixtures of polyethylene glycols of various molecular weights, and fatty acid esters of polyethylene glycol.

For administration by inhalation, the compounds of (or for use with) the invention (e.g. an antigenic particle of the invention) are typically delivered in the form of an aerosol spray from pressured container or dispenser which contains a suitable propellant, e.g., a gas such as carbon dioxide, or a nebuliser.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions can be formulated into ointments, salves, gels, or creams as generally known in the art.

In certain embodiments, the pharmaceutical composition is formulated for sustained or controlled release of a compound of (or for use with) the invention (e.g. an antigenic particle of the invention). Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

Exemplary unit dosage forms for pharmaceutical compositions comprising the antigenic particles of the invention are tablets, capsules (e.g. as powder, granules, microtablets or micropellets), suspensions or as single-use preloaded syringes. In certain embodiments, kits are provided for producing a single-dose administration unit. The kit can contain both a first container having a dried active ingredient and a second container having an aqueous formulation. Alternatively, the kit can contain single and multi-chambered preloaded syringes.

As mentioned above, preferred pharmaceutical compositions are vaccine compositions. As used herein, the term "vaccine composition" refers to an immunogenic composition which, when administered to a subject, elicits protective immunity against an antigen, in this case the antigen represented by the immunogenic compound. Antibodies produced in accordance with the invention may also be used as therapeutics or passive immunization.

Furthermore, the vaccine composition may include one or more adjuvants. As used herein the term "adjuvant" refers to a compound that, when used in combination with a specific antigenic particle in a formulation, will augment or otherwise alter or modify the resultant immune response. Modification of the immune response can include intensification or broadening the specificity of either or both antibody and cellular immune responses. Modification of the immune response can also mean decreasing or suppressing certain antigen-specific immune responses.

Examples of known adjuvants include complete Freund's adjuvant (a non-specific stimulator of the immune response, containing killed Mycobacterium tuberculosis), incomplete Freund's adjuvants and aluminium hydroxide adjuvant. Other known adjuvants include granulocyte macrophage colony-stimulating factor (GM-CSF), Bacillus Calmette-Guerin (BCG), aluminium hydroxide, Muramyl dipeptide (MDP) compounds, such as thur-MDP and nor-MDP, muramyl tripeptide phosphatidylethanolamine (MTP-PE), RIBI's adjuvants (Ribi ImmunoChem Research, Inc., Hamilton Mont.), which contains three components extracted from bacteria, trehalose dimycolate (TDM) and cell wall skeleton (CWS) in a 2% squalene/Tween 80 emulsion. MF-59, Novasomes®, major histocompatibility complex (MHC) antigens are other known adjuvants.

In some embodiments the vaccine composition may include two, three, four, five, six, seven, eight, nine or more antigenic particles, each comprising a difference immunogenic compound derived from one or more antigens.

The herein provided substances, compositions and systems of the invention are preferably for use in the prevention, management, and/or treatment of a medical condition. Hence, the invention relates to a use of the herein disclosed substances, compositions or systems for the manufacture of a medicament of the prevention, management, and/or treatment of a medical condition. Furthermore, the invention relates to a method for prevention, management, and/or treatment of a medical condition, the method comprising the administration of an effective amount of a substances, compositions or system of the invention to a subject in need of such a treatment.

A medical condition which can be prevented, managed or treated in accordance with the invention is in preferred embodiments an addiction to a dependence-causing substance. In this embodiment the immunogenic compound of the antigenic particle is the dependence-causing substance. Without being bound to theory it is expected that the antigenic particles of the invention provide an improved vaccination strategy leading to a strong immune response in a subject and thereby antibodies produced in the subject in response to the antigenic particle will serve as "sponges" to neutralise the dependence-causing substance in the event the subject consumes such substance during a fall-back to the addictive behaviour. Thereby, the vaccination strategy of the invention reduces the positive sensation of the dependence-causing substance and helps the subject to overcome the addiction.

A medical condition which can be prevented, managed or treated in accordance with the invention is in other preferred embodiments an adverse or fatal event caused by drug adulterant. Drug adulterants are substances consumed together with a preparation of a drug of abuse, but often lead to severe overdosing and not-rarely serious adverse effects and even death of the subject. In this embodiment the antigenic particle of the invention includes an immunogenic compound inducing an immune response against a common adulterant in order to protect the abuser from an overdose. Preferably in such a treatment the subject is administered also an antigenic particle of the invention specific for the drug of abuse to which the subject has an addiction.

In further embodiments the invention pertains to a medical condition which is an infectious disease, and wherein the immunogenic compound of the antigenic particle is an immunogenic compound or sequence derived from the infectious organism causing the infectious disease. In preferred aspects the disease is malaria. In other preferred aspects and embodiments, the infectious disease is a viral disease such as a disease caused by a Corona virus, such as Covid-19. With respect to malaria and Covid-19, known and preferred antigens are well known to the skilled person. For example, the immunogen is a compound derived from the viral or plasmodium surface.

Other embodiments of the invention pertain to cancer, and wherein the immunogenic compound of the antigenic particle is a compound associated with or specific for a cancer cell of the cancer.

Other embodiments of the invention pertain to the creation of "slow release depots" (SRDs) for the application of chemotherapeutic agents. SRDs are antibodies or any product of vaccination that bind to specific chemotherapeutic compounds and alter the pharmokinetics or other properties of the treatment for useful purposes (such as reduction of toxicity, prolongation of half-life, alternative tissue tropism, etc.). An example is anti-cancer chemotherapeutic agents (platinum, Adriamycin, etc.) and the generation of antibodies or immune reactions from vaccination that allow for the capture and "slow release" of the chemotherapeutic agents for therapeutic or other useful purposes. Other examples include chemotherapeutics for infectious diseases or allergies for which immunological SRDs provide useful approaches. SRDs for compounds used in industrial applications would also be an embodiment of this invention.

An additional embodiment of the invention pertains to aspects of neurodegenerative diseases and antibodies or any product of vaccination against specific proteins. Examples include: Alzheimer's disease (Amyloid beta (Ab) peptide, Tau), Parkinson's disease (a-Synuclein), Multiple Tauopathies (Tau protein, microtubule-associated), Huntington's disease (Huntingtin with or without tandem glutamine repeats), Amyotrophic lateral sclerosis (Superoxide dismutase 1), Spongiform encephalopathies (Prion proteins), Familial amyloidotic polyneuropathy (Transthyretin, wild-type and mutant forms), small peptides like those associated with migraines), and any possible peptide or small molecule targets that are deemed useful, including but not limited to industrial, agricultural, research or diagnostic purposes.

In general, anything for which an antibody or generalized immune response has shown or could reasonably be expected to potentially show usefulness in a medical, diagnostic or industrial settings qualifies as an embodiment of this invention.

The medical use of the antigenic particle of the invention usually involves the administration of the antigenic particle to a subject in need of the prevention, management, and/or treatment of the medical condition, for example in the form of a vaccine composition as described herein before.

Yet another aspect of the present invention then pertains to a method for the generation of an antibody which is capable of binding to an immunogenic compound, the method comprising the steps of providing an antigenic particle according to the invention, wherein the immunogenic compound of the antigenic particle is the immunogenic compound, or immunogenic parts thereof, the antibody to be generated is capable to bind to; immunizing an antibody-producing human or non-human animal with the antigenic particle; isolating from the immunized human or animal immune cells producing antibodies against said immunogen, and optionally, isolating from said cells said so generated antibodies.

In context of the herein disclosed invention the non-human animal is preferably selected from mouse, rabbit, camel, chicken goat, rat, dog, cat, monkey, hamster or other mammals.

Yet a further aspect then pertains to a method of vaccinating a subject in need of an enhanced immune response specific for an immunogenic compound, the method comprising administering to the subject an amount of an antigenic particle according to the invention which is sufficient to induce an immune response in the subject against the immunogenic compound, wherein the immunogenic compound comprised in the antigenic particle is identical to or is an immunogenic part of, the immunogenic compound for which the enhanced immune response is specific. Preferred is a method for preventing, managing or treating a medical condition, and wherein the immunogenic compound is associated with the medical condition.

Such adverse condition is for example an addiction, and the antigenic compound is an addiction causing substance.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

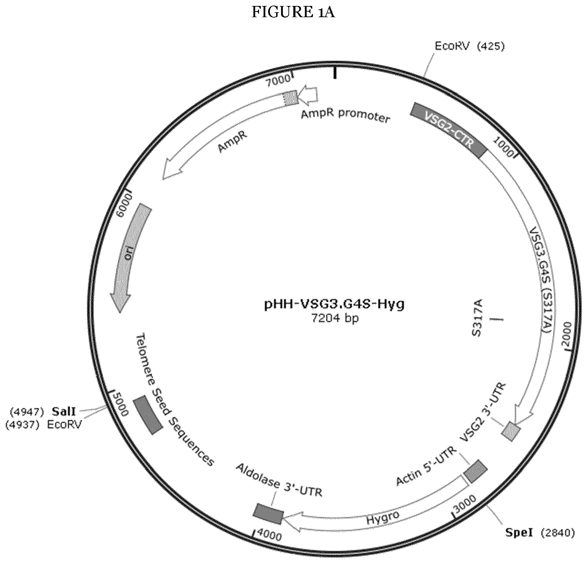

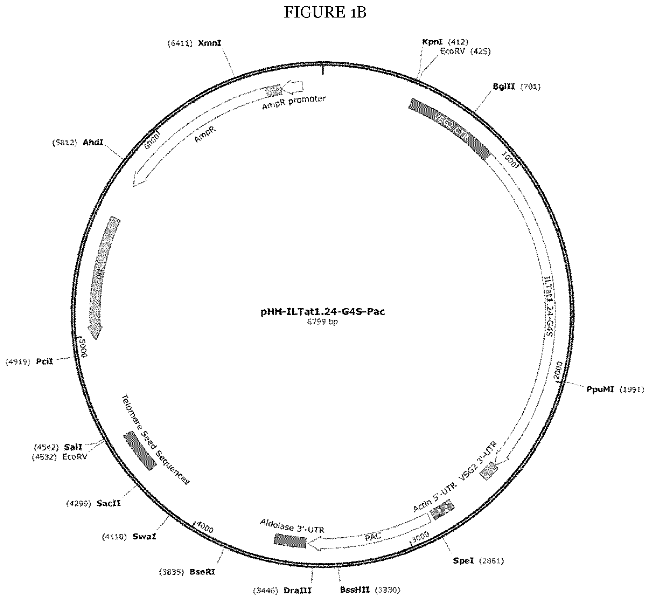

**Figure 1A** **and** **B****. Map of plasmids used for VSG engineering.** Sequences of pHH-VSG3.G4S-Hyg and pHH-ILTat1.24-G4S-Hyg plasmids are provided in SEQ ID NO: 6 and SEQ ID NO: 7 respectively. **(A)** pHH-VSG3.G4S-Hyg Plasmid Size: 7204 bp. VSG2-CTR (VSG2 Co-transposed Region): 429-878; VSG3.G4S (S317A): 879-2453; VSG2 3'-UTR (VSG2 3'-Untranslated Region): 2454-2533; Actin 5'-UTR (Actin 5'-Untranslated Region): 2727-2834; Hygro (Hygromycin Resistance Gene): 2854-3879; Aldolase 3'-UTR (Aldolase 3'-Untranslated Region): 3885-4033; Telomere Seed Sequences: 4715-4915; Ori (Bacterial Origin of Replication): 5385-5973; AmpR (Ampicillin Resistance Gene, Beta-lactamase): 6144-7004 (Reverse Complement); AmpR Promotor (Ampicillin Resistance Gene Promoter, Beta-Lactamase Promoter): 7005-7109 (Reverse Complement); **(B)** pHH-ILTat1.24-G4S-Hyg Plasmid Size: 7219 bp. VSG2-CTR (VSG2 Co-transposed Region): 429-878; ILTat1.24-G4S: 879-2468; VSG2 3'-UTR (VSG2 3'-Untranslated Region): 2469-2548; Actin 5'-UTR (Actin 5'-Untranslated Region): 2742-2849; Hygro (Hygromycin Resistance Gene): 2869-3894; Aldolase 3'-UTR (Aldolase 3'-Untranslated Region): 3900-4048; Telomere Seed Sequences: 4730-4930; Ori (Bacterial Origin of Replication): 5400-5988; AmpR (Ampicillin Resistance Gene, Beta-lactamase): 6159-7019 (Reverse Complement); AmpR Promotor (Ampicillin Resistance Gene Promoter, Beta-Lactamase Promoter): 7020-7124 (Reverse Complement).

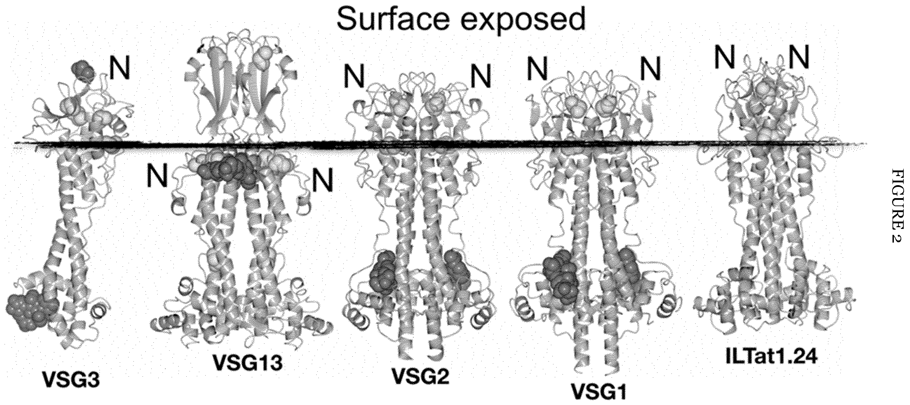

**Figure 2****. Crystallographic studies help to determine the accessibility of the VSG N-terminus to the sortase enzyme as illustrated using 5 examples** (all published except VSG13). "N" denotes the location of the free N-terminus for each protein. The line denotes a roughly estimated general location of surface-exposed residues (the "top" of the VSG coat, what would be accessible to the sortase enzyme). While many VSGs can be engineered to accept tags through sortase-based conjugation (and the inventors have already done this for VSG2, VSG3 and ILTat1.24 as discussed later), not all can be (for example the N-terminus in VSG13 is far more buried and may not be sufficiently accessible). Structural biology presents a very useful pre-screening of VSGs to uncover many surface elements and other architectural features that inform the choices of VSGs (e.g., the discovery of the O-linked sugar on VSG3 that led the inventors to work with a specific serine to alanine mutant, S317A, to remove it from the surface).

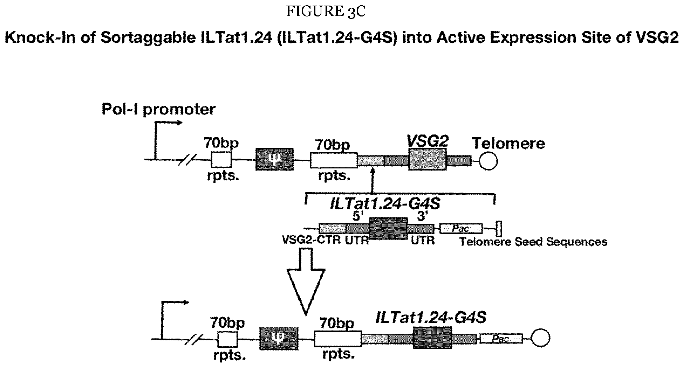

**Figure 3****. Knock-in strategy of sortaggable VSGs into the genome of *Trypanosoma brucei.*** A to C show the genetic cloning strategy to express engineered VSG2, VSG3 and ILTat1.24 from the active expression site of endogenous VSG (replacing wild-type VSG2).

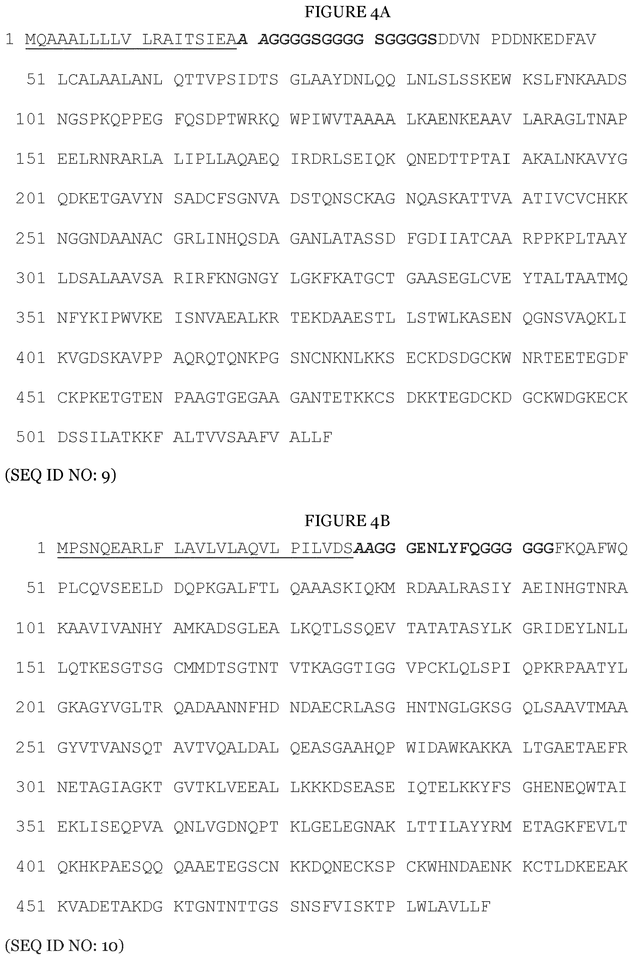

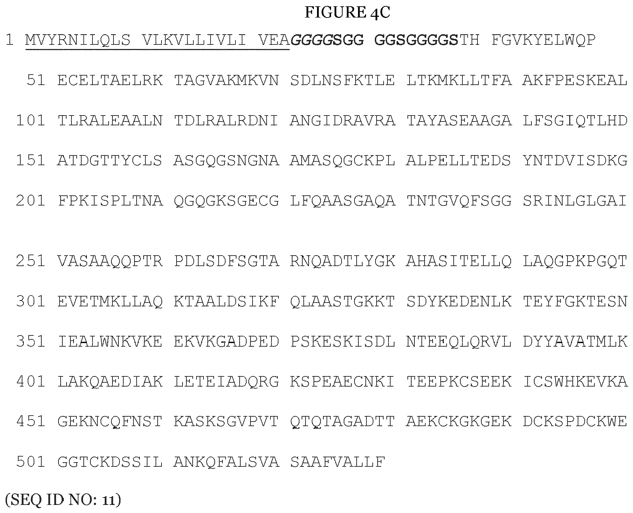

**Figure 4****. Amino acid sequences of the sortaggable VSG proteins. A. Amino acid sequence of the sortaggable VSG3.G4S (S317A) protein.** The signal peptide is underlined. The mature, sortaggable VSG3 is derived from a more antigenic mutant of wild-type VSG3 (S317A). VSG3-G4S will initiate with the di-Alanine (in bold and italic). This dipeptide will be the acceptor of the sortase A reaction (and will accept any moiety N-terminally linked to the peptide sequence LPSTGG). The extension of the N-terminus (by addition of the (G₄S)₃ peptide linker, in bold), is crucial for the ability of sortase A to access the di-Alanine. **B. Amino acid sequence of the sortaggable VSG2 protein (VSG2-1DK).** The signal peptide is underlined. The mature, sortaggable VSG2-1DK will initiate with the di-Alanine (in bold and italic). This dipeptide will be the acceptor of the sortase A reaction (and will accept any moiety N-terminally linked to the peptide sequence LPSTGG). The extension of the N-terminus (by addition of a linker peptide consisting of a TEV protease cleavage site flanked by poly-Glycine, in bold) is crucial for the ability of sortase A to access the di-Alanine. **C. Amino acid sequence of the sortaggable ILTat1.24 protein (ILTat1.24-G4S).** The signal peptide is underlined. The mature, sortaggable ILTat1.24-G4S will initiate with tetra-Glycine (shown in bold and italic). This tetra-Glycine will be the acceptor of the Sortase A reaction (and will accept any moiety N-terminally linked to the peptide sequence LPSTGG). The extension of the VSG N-terminus (by addition of the (G4S)2 peptide linker, in bold), is crucial for the ability of Sortase A to access the tetra-Glycine.

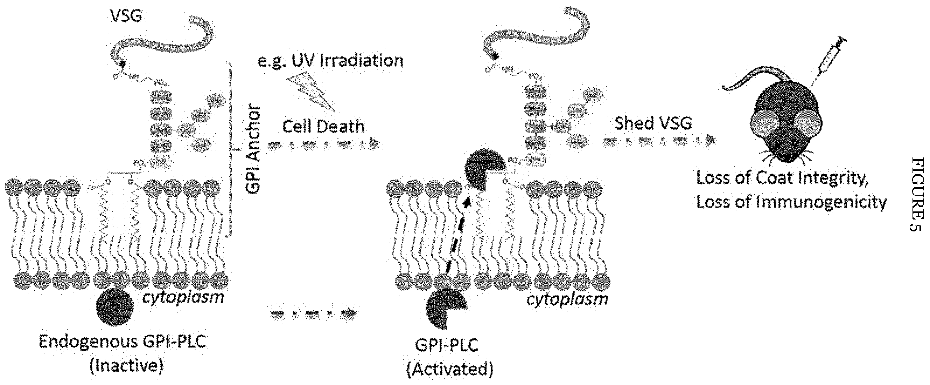

**Figure 5****. Wild-type *T. brucei* sheds its VSG coat upon death. A.** Cartoon of the process showing that GPI-PLC is activated upon cell death. **B.** The location of GPI anchor cleavage site is shown. Cleavage of the GPI anchor releases (sheds) VSG from the coat and the coat-less trypanosome disintegrates through osmotic pressure (and consequently losing its immunogenic properties).

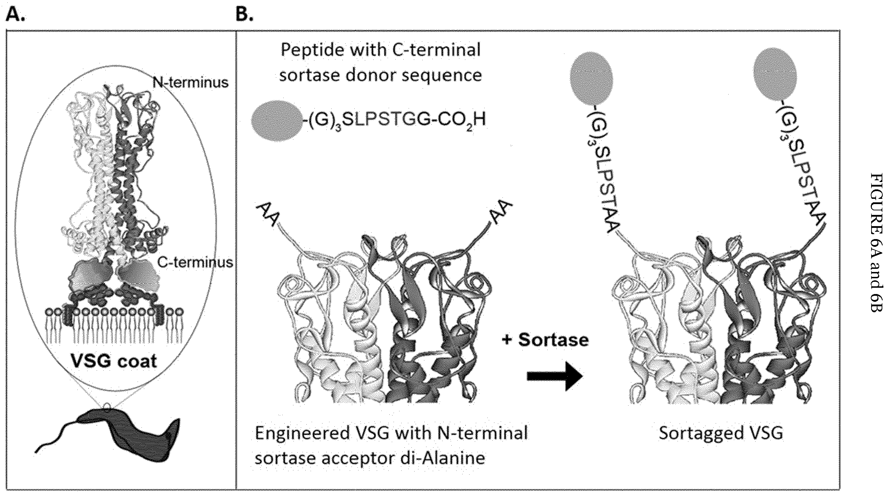

**Figure 6****. Illustration of the overall method of sortagging the VSG coat of a Trypanosome. A.** Visualization of a VSG protein homodimer embedded in the membrane of a Trypanosome via a glycophosphatidylinositol (GPI) anchor. Bottom: whole trypanosome. Top: Zoom on one VSG protein homodimer. **B.** Illustration of the sortagging reaction: modified VSG proteins (with an N-terminal di-Alanine), and small molecules (oval) linked to the sortase donor sequence LPSTGG, are covalently linked via a sortase reaction.

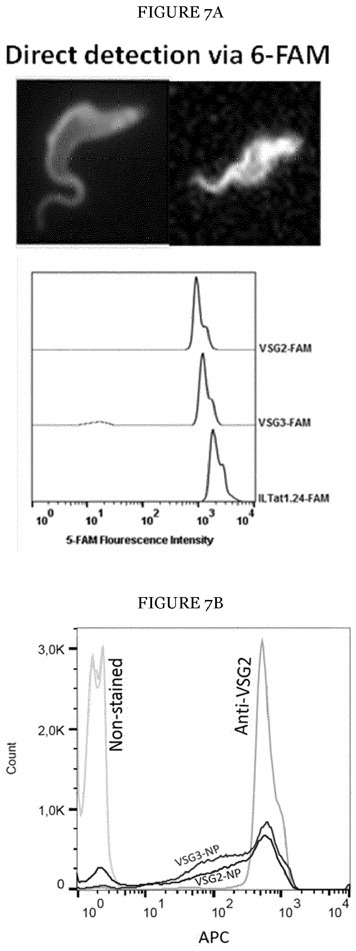

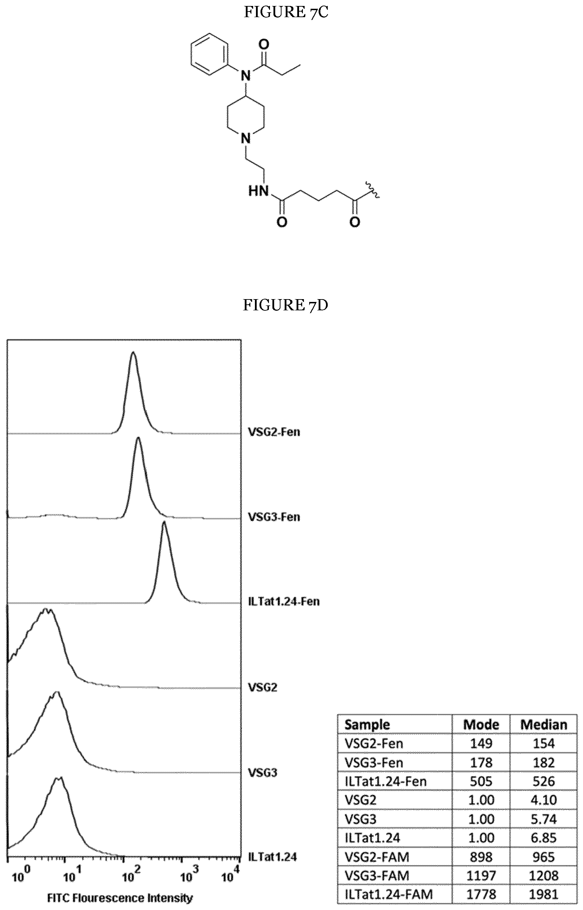

**Figure 7****. Methods to detect Sortagging efficiency. A.** Top image: Sortagging of VSG2-1DK (left) and VSG3-G4S (S317A) (right) detected via direct fluorescence (6-FAM). Fluorescent microscopic images of a *T. brucei* cell are shown at top (left: sortagged VSG2-1DK, right: sortagged VSG3-G4S). Bottom image: The 6-FAM sortagged VSGs were also analyzed by flow cytometry analysis using FACSCalibur. **B.** Sortagging of VGS2-iDK and VSG3-G4S (S317A) detected via FACS analysis of Trypanosomes using a monoclonal antibody against a small-molecule moiety (4-hydroxy-3-nitrophenylacetyl, abbreviated as NP here) followed by staining with an Allophycocyanin (APC)-conjugated mouse monoclonal IgG antibody (B1-8 clone, Abcam). **C.** A derivatized fentanyl hapten was chemically synthesized and conjugated to the N-terminus of a peptide containing a C-terminal sortase A donor sequence (fentanyl-GGGSLPSTGG, where fentanyl-conjugated compounds are alternatively denoted "Fen-" or "-Fen"). The peptide carrying the fentanyl hapten was conjugated to three different genetically modified VSGs (VSG2, VSG3 and ILTat1.24) using sortase A as described before. Chemical synthesis process of the fentanyl hapten has been described by M. D. Raleigh et al., J. Pharmacol. Exp. Ther. 368:282-291, 2019, and it has been adapted for sortase-mediated conjugation here. **D.** After sortase A-mediated conjugation of the fentanyl hapten to VSGs, a mouse monoclonal antibody against fentanyl (provided by M. Pravetoni, University of Minnesota) was conjugated to FITC using a kit (Abcam, ab102884) and used to stain the Sortagged VSGs followed by flow cytometry analysis using FACS-Calibur. Non-tagged VSGs were used as control for background staining. Below the graph: The mode and median of the data sets are shown. In both fentanyl and FAM conjugations, ILTat1.24 outperformed VSG2 and VSG3. Also, Sortagging efficiency of VSG3 was moderately higher than VSG2.

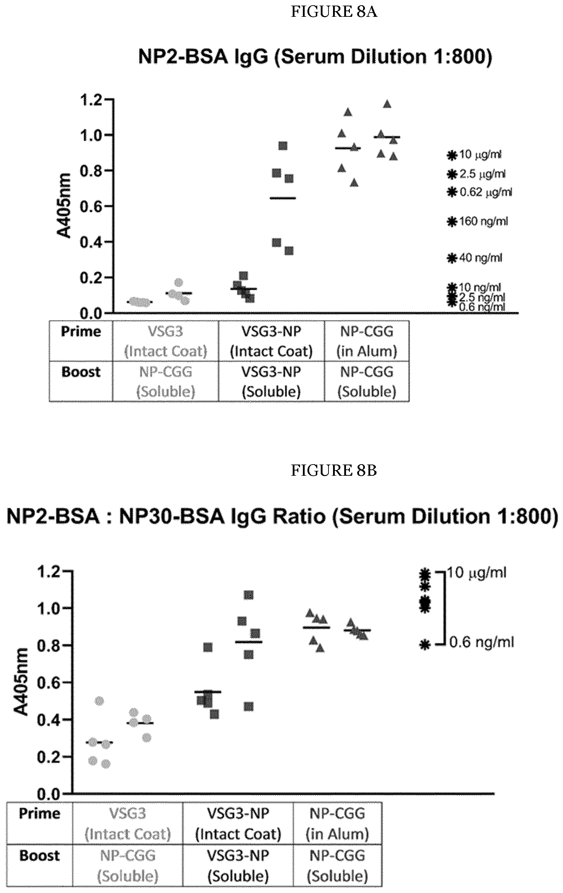

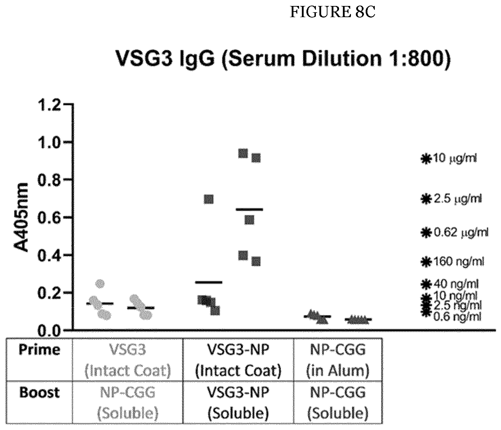

**Figure 8****.** Comparison of antibody responses to the small molecule 4-hydroxy-3-nitrophenyl acetyl (NP): Immunization with NP-labeled *T. brucei vs.* the "gold standard" hapten-carrier conjugate (i.e. NP-conjugated chicken gamma-globulin (NP-CGG) in Alum adjuvant). Five 6-8 weeks old female C57BL/6J mice per group were primed at day 0,3 and 30 with intact VSG3(S317A), or VSG3-NP coats (i.e. U.V-irradiated intact *T. brucei* cells expressing sortaggable VSG3(S317A), either tagged or not tagged with NP hapten, without adjuvant) or with NP-CGG in Alum adjuvant. These mice received a soluble VSG3(S317A)-NP (in PBS, without adjuvant) booster at day 70 (or were boosted with soluble NP-CGG, in PBS without adjuvant, for the control group). **A.** The priming immunization with VSG-NP on intact trypanosomes followed by boosting with soluble VSG3(S317A)-NP, shows a clear IgG recall response and results in the generation of substantial IgG titers to the small molecule hapten NP. Titers were measured before and after boost and are shown at serum dilution 1:800. An anti-NP hapten monoclonal IgG (B1-8 clone, Abeam) was serially diluted (4-fold) to cover a range of concentrations from 10 µg/ml to 0.6 ng/ml. Immunization with the conjugated trypanosome coats results in high IgG titers to NP (average ∼500 µg/ml). Furthermore, the fact that soluble VSG3-NP (in PBS, without adjuvant) induced a secondary IgG response strongly indicates that immunization with NP-conjugated VSG coats can induce a memory B cell response. While mean titers raised against NP-CGG in Alum are somewhat higher, boosting with soluble NP-CGG (in PBS, without adjuvant) did not induce a robust secondary response, which indicates lack of a memory B cell response after priming with NP-CGG in Alum. **B.** Immunization using VSG-NP on intact trypanosome coats followed by boosting with soluble VSG3(S317A) NP results in high affinity IgG (as defined by NP2/NP30 IgG titer ratios). Increase in NP2-BSA/NP30-BSA IgG ratio in VSG3-NP group indicates affinity maturation, a hallmark of memory B cell (recall) response. Immunization with the "gold standard" hapten-carrier conjugate (NP-CGG) in Alum provides no increase in affinity of anti-NP IgG antibodies. **C.** Immunization with VSG-NP on intact trypanosome coats followed by boosting with soluble VSG3(S317A)-NP also yields anti-VSG3 (anti-carrier) antibodies, but those are of a comparable magnitude (in µg/ml) to antisera raised to the NP hapten (as quantified by serial dilutions of an anti-VSG3 mouse monoclonal IgG (11D6 clone). Additional data demonstrate a lack of immunological cross-reactivity between the VSG2 and VSG3 carriers (not shown).

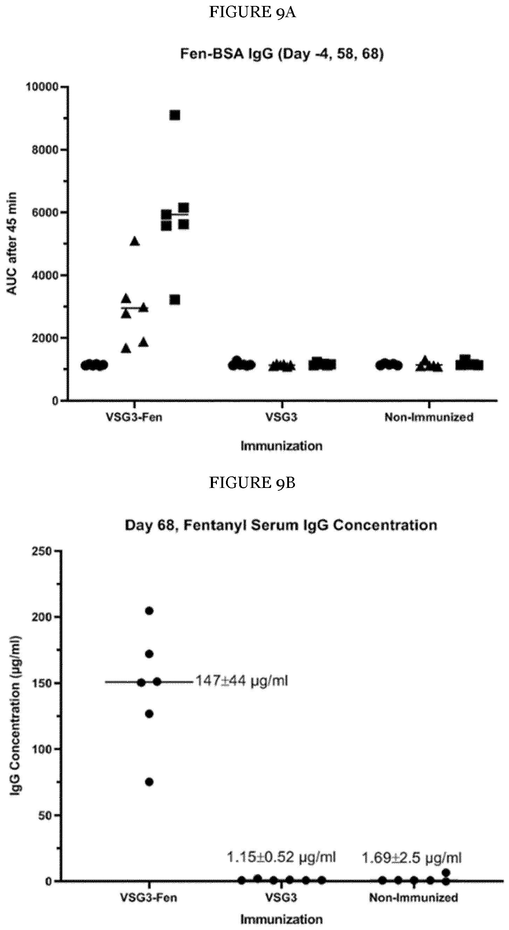

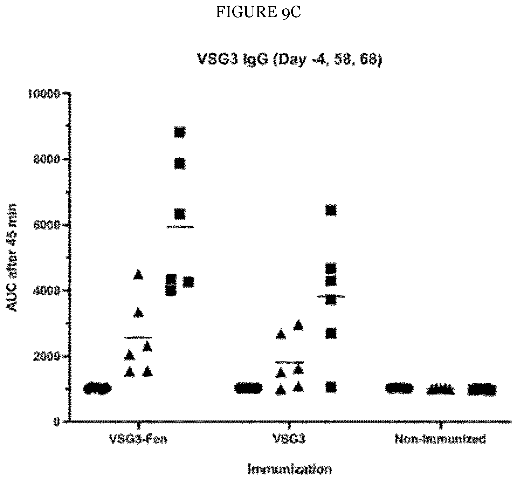

**Figure 9****. Antibody responses to fentanyl elicited by Fen-labeled *T. brucei* achieve high titers and memory (recall).** ELISA measurement of serum IgG against fentanyl hapten and VSG3 carrier protein are shown. **A.** A tetra-Glycine peptide carrying an N-terminal fentanyl hapten (Fen-G₄) was synthesized as described before. The Fen-G₄ peptide was conjugated to BSA as a heterologous carrier protein and used to coat 96-well ELISA plates at 10 µg/ml. Five 6-8 weeks old female C57BL/6J mice per group were primed at day 0 and 30 with intact VSG3(S317A), or VSG3(S317A)-Fent coats (i.e. U.V-irradiated intact *T. brucei* cells expressing sortaggable VSG3(S317A), either tagged or not tagged with Fen-hapten, without adjuvant) via subcutaneous injection. These mice then received a soluble VSG3(S317A)-Fent (in PBS, without adjuvant) booster at day 60. Serum samples tested included the pre-immune (day -4), 2 days before (day 58) and 8 days after (day 68) 1 boost with soluble VSG3(S317A)-fentanyl protein. Anti-fentanyl IgG in sera was detected using an anti-mouse HRP conjugate (1:3000) followed by addition of ABTS substrate and H₂O₂ prepared in citrate-phosphate buffer pH 4.2. Absorption of the samples were measured after 45 min at A405 nm using an ELISA reader (Tecan, Infinite M1000 Pro). **B.** A mouse monoclonal antibody against Fen-hapten was serially diluted to make a calibration curve in order to quantify IgG concentration in serum samples. Mean ± standard deviation of 6 mice per group are shown. **C.** Similarly, 96-well plates were coated with FPLC-purified VSG3(S317A) protein at 5 µg/well to measure serum IgG against VSG3(S317A) carrier protein. Area under curve (AUC) after 45 min was calculated by GraphPad Prism. The circles, triangles and squares indicate sera at day -4, 58 and 68 respectively. Immunization with the conjugated trypanosome coats results in high IgG titers to fentanyl (average ∼150 µg/ml). Furthermore, the fact that soluble VSG3(S317A)-Fen (in PBS, without adjuvant) induced a secondary IgG response strongly indicates that immunization with Fen-conjugated VSG coats can induce a memory B cell response.

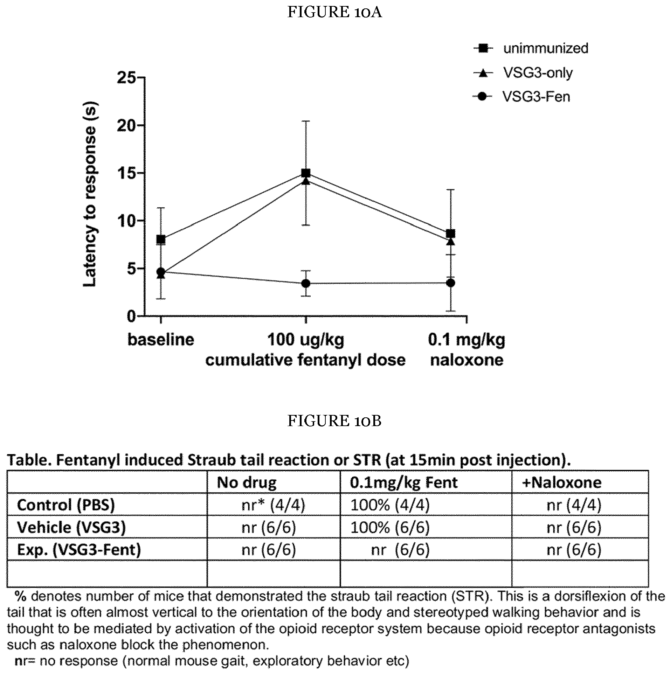

**Figure 10****. Mice immunized with Fentanyl-haptenated *T. brucei* are protected from intoxication. A.** Analgesic activity. Analgesic activity was tested by using the hotplate antinociception assay as described by Cox and Weinstock (1964). Fentanyl effect on hotplate antinociception was tested in unimmunized mice, in mice immunized with carrier only (VSG3(S317A)-only) or in mice immunized with haptenated VSG3(S317A)-Fen. In all cases mice were dosed with a cumulative fentanyl concentration of 0.1 mg/kg (s.c.). Fentanyl was administered subcutaneously every 15-30 minutes at increasing doses and the dose listed is the cumulative dose received. Hotplate antinociception was measured 15 minutes after the final fentanyl dose. Naloxone (0.1 mg/kg, s.c.) was administered 15 minutes after the final fentanyl dose. The effect of fentanyl is shown as latency to response. Fentanyl increased the latency to response after a cumulative dose of 0.1 mg/kg in unimmunized and VSG3-only immunized mice, compared with their baseline values. Naloxone completely reversed fentanyl-induced antinociception in both groups. Mice immunized with VSG3(S317A)-Fen did not show an increase in latency to response, compared to baseline, thus demonstrating that those mice did not get intoxicated by fentanyl at the same dose as the controls. Mean ± standard deviation of 5 (unimmunized) or 6 mice per group are shown. **B.** Straub tail reaction (STR) measured per mouse per group. % denotes number of mice that demonstrated the Straub tail reaction, a dorsiflexion of the tail that is often almost vertical to the orientation of the body or curling back over the animal and stereotyped walking behavior (Bilbey et al, 1960). This phenomenon was first described as a response to opiates in mice (Straub, 1911), and is thought to be mediated by activation of the opioid receptor system because opioid receptor antagonists such as naloxone block the phenomenon (Aceto et al, 1969; Nath et al., 1994; Zarrindast et al, 2001).

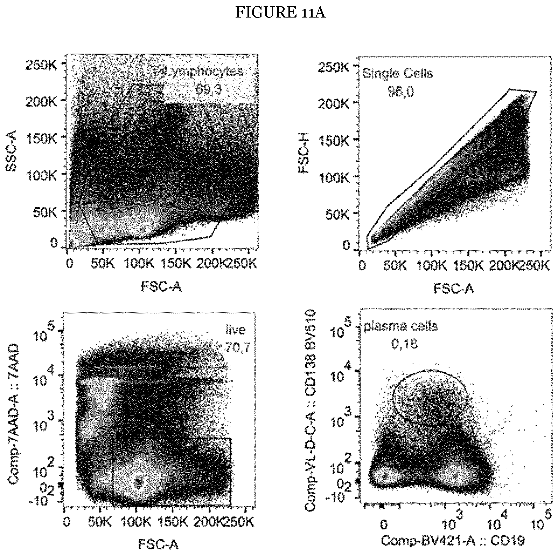

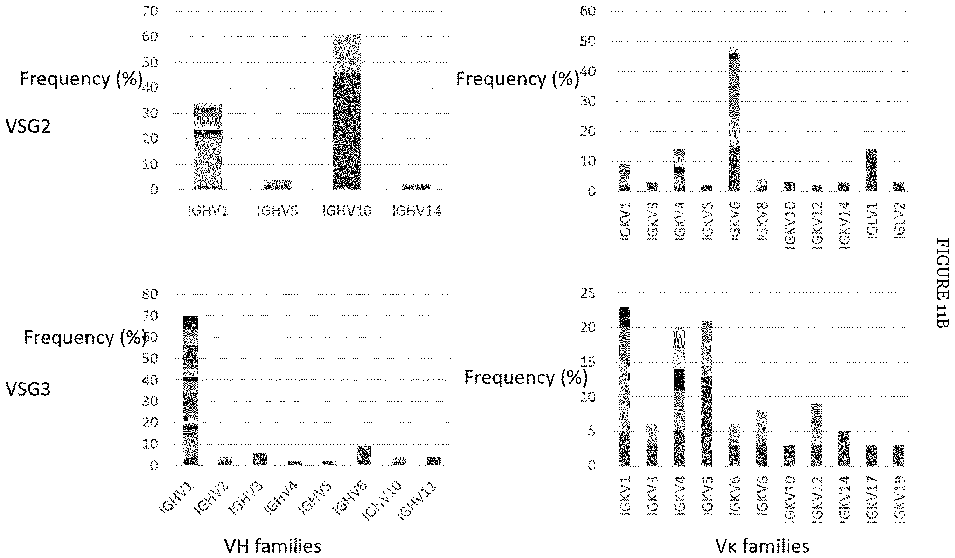

**Figure 11****. Each distinct VSG coat elicits a unique subset of B cell specificities (thus, a unique B cell repertoire).** Trypanosomes expressing either VSG2 trypanosome coats or the VSG3(S317A) version used in the fentanyl vaccination experiments (a more immunogenic form of VSG3 with serine 317 mutated to alanine) elicit distinct repertoires in C57BL/6 mice. **A.** Gating strategy for the isolation of plasma cells. Lymphocytes were isolated from spleens and analyzed on a LSR II instrument for plasma cells. Plasma cells were then isolated (single cell sorted) using an ARIA II cell sorter. Cells were stained using an anti-mouse CD19-BV421 and anti-mouse CD138-BV510 antibodies. 7AAD was included in all stainings to exclude dead cells. The data were analyzed using FlowJo v10 software. Ig gene cloning was performed as described before (Tiller et al., 2008). In brief, cDNA of each single cell was generated using random hexamer primers. Ig heavy and corresponding Ig kappa or Ig lambda light chain gene transcripts were amplified using a semi-nested PCR strategy (Tiller et al., 2008). Amplicons were Sanger sequenced and analyzed using NCBI IgBlast. **B.** V gene repertoires elicited by VSG2 or VSG3(S317A) coated trypanosomes. Histograms summarize the VH and Vκ gene family usage in Ig gene transcripts isolated from plasma cells from C57BL/6 mice exposed to VSG2 or VSG3(S317A) coated trypanosomes (59 and 53 distinct Ig transcripts respectively). Sequences were obtained from 4 and 8 different VH gene families for VSG2 and VSG3(S317A), respectively, and 11 Vκ gene families. Within one bar the different shades of gray show the distribution of different family members within the respective family. The VH10 family, followed by the VH1 family (the largest VH family) was preferably expressed by the majority of plasma cells isolated from the VSG2 mice whereas only very few plasma cells expressing the VH10 family were isolated from the VSG3(S317A) mice. For VSG3 mice, most plasma cells expressed the VH1 family. In the VSG2 mice plasma cells mainly expressed the VK6 family, whereas in VSG3(S317A) mice the Vk family usage was more divers (but mainly VK1, VK4 and VK5). The analyzed sequences clearly demonstrate that the two different VSGs elicit a different VH and Vk family repertoire in plasma cells. This specificity allows one to design optimal vaccination strategies using distinct VSG coat platforms, tailored to the "needs" of specific epitopes. For example, this method can be used to expand an infrequently present B cell that is nevertheless required to be clonally expanded to produce an optimal response against a specific target.

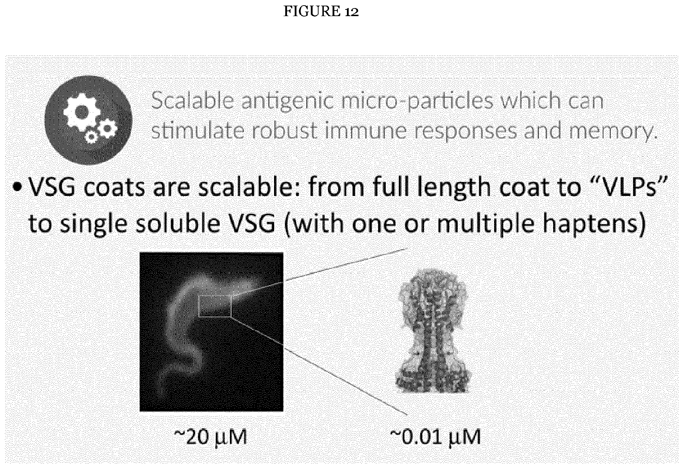

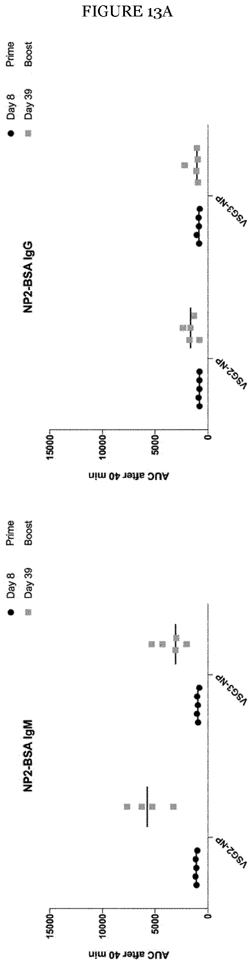

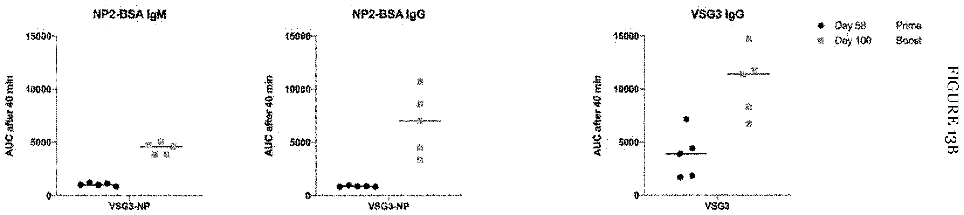

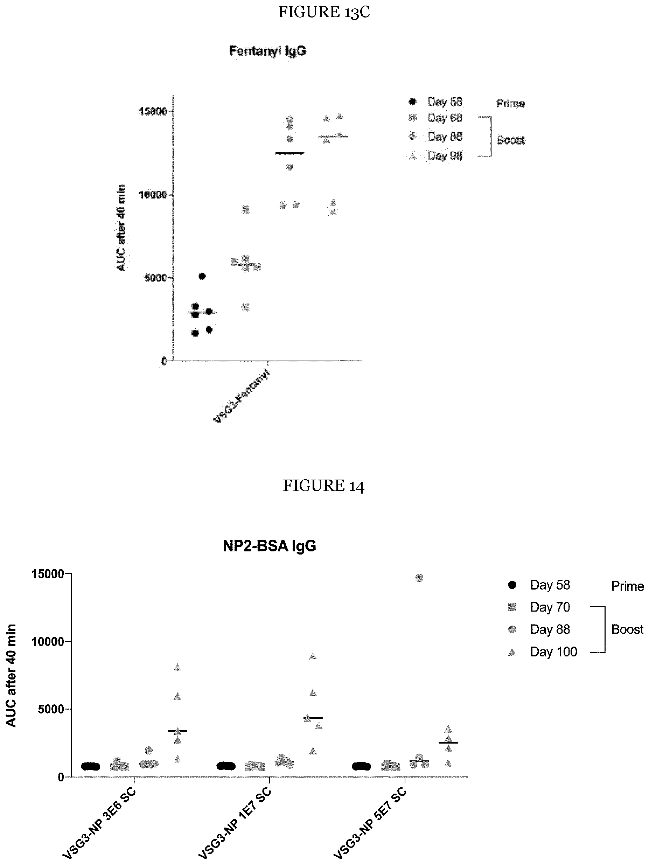

**Figure 12****:** Illustrates the two types of particles that can be generated from trypanosomes. Immunization with large, intact, inactivated particles generates (Fig. 13) a traditional T-independent response. We have discovered that a subsequent boost with soluble VSG particles (i.e. the same material as the initial immunogen but now purified away from the coats in soluble form) is uniquely capable of generating a recall (memory) response together with very high antigen specific IgG titers.
**Figure 13****:** shows immunization with haptenated trypanosomes generates high titers of hapten-specific antibodies, and memory. **A.** Mice primed twice with the NP-haptenated entire VSG coat of trypanosomes followed by a boost with the same moiety (NP-haptenated entire VSG coat of trypanosomes) show a predominantly serum IgM response against NP and no IgG response. **B.** Mice primed twice with the NP-haptenated entire VSG coat of trypanosomes followed by a boost with the same moiety but now in soluble form show high serum IgG antibody titers again NP and memory. IgG titers are equivalent in proportion against both the carrier as well as the hapten. **C.** IgG titers elicited by our prime-boost combination are long lasting (e.g. still increasing even one month after the first boost, from Day 68 to Day 98). Data is shown for two different trypanosome strains expressing either VSG2 or VSG3. Data is presented as area under curve (AUC) from a serum dilution range from 1:100 to 1:12800. Each dot represents one mouse (n=5 mice per immunogen).
**Figure 14****:** Mice were primed and boosted with either 3E6, 1E7 or 5E7 NP-haptenated VSG3 coats subcutaneously. Mice receiving the highest dose of 5E7 NP-haptenated VSG3 coats showed the lowest NP specific serum IgG titers after the last bleed on Day 100. Thus dose escalation (increase) is not necessarily beneficial and potentially even detrimental to antigen-specific serum IgG titers.

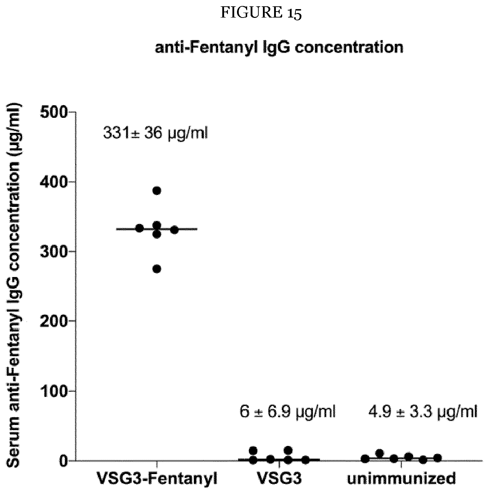

**Figure 15****:** The specific prime-boost combination elicits high serum antibody titers against the hapten (here quantified for fentanyl haptenated to VSG3 which represent about 15% of total IgG in a mouse (2.38 mg/ml total IgG in a mouse) mouse (Klein-Schneegans et al., 1989)).

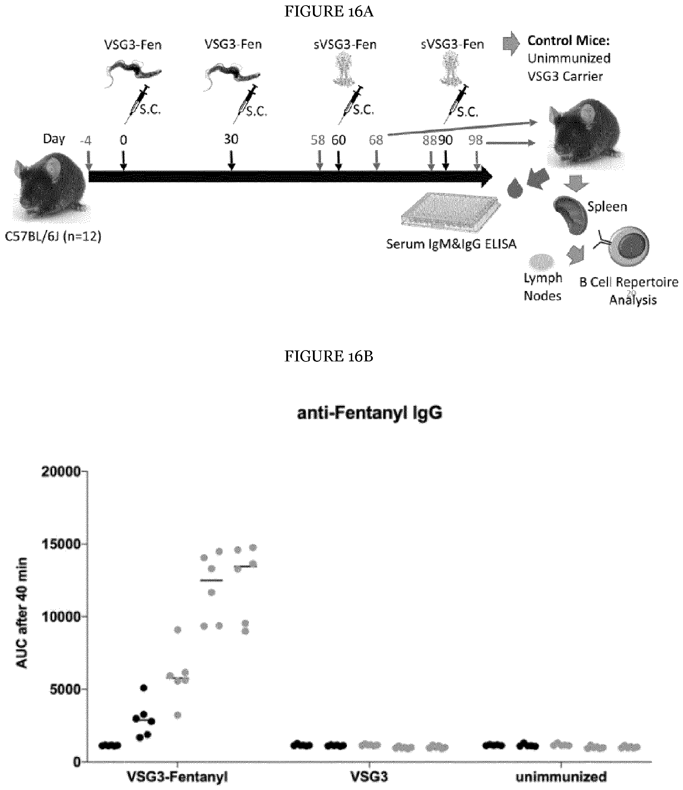

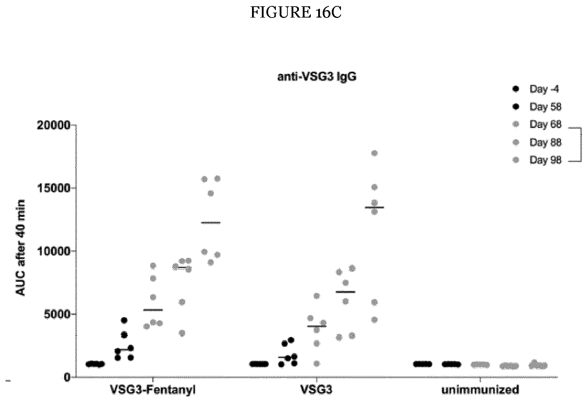

**Figure 16****:** Immunization with fentanyl-haptenated trypanosomes generates high titers of anti-Fen antibodies, and memory. **A.** Experimental timeline. **B.** ELISA titers presented as "area under curve" (AUC) for a dilution range from 1:100 to 1:12800. The ELISA plate is coated with Fentanyl-BSA (Fen-BSA). The immunogen (Fen-VSG3, VSG3 carrier alone or no immunogen) is noted on the x-axis). Each dot represents antiserum from 1 mouse (n=6 mice per immunogen) (4 time points per immunogen starting with pre-bleed - collected at day -4 - up to post-2nd boost - collected at day 98 - as per the timeline in A. **C.** ELISA titers presented as AUC for a dilution range from 1:100 to 1:12800 of antisera against the carrier. The ELISA plate is coated with VSG3. The immunogen (Fen-VSG3, VSG3 carrier alone or no immunogen) is noted on the x-axis.

The sequences show:

SEQ ID NO: 1 >Tb427VSG-1 : GB Accession X56761.2 | Trypanosoma brucei brucei |
Lister427 | variant surface glycoprotein MITat 1.1 (Lister 427-1|Not fully assembled by me) |
Source=GenBank download 170507 | Protein length=492

MATGRAKNTKWARWLSTAGLIIVVTLPATTMAAERTGLKATAWKPLCKLTTELSKVSG
EMLNEGQEVISNIQKIKAAEYKVSIYLAKNPETQALQQLTLLRGYFARKTNGGLESYKTMGLAT
QIRSARAAAYLKGSIDEFLNLLESLKGGSENKCLVTTNADTAATRRETKLDDQECALSMPETKP
EAATRTELTQTGYPNLQHGGGGTANTFQPTTSTGTCKLLSGHSTNGYPTTSALDTTAKVLAGY
MTIPNTQVEATLANMQAMGNGHKATAPAWHEAWEARNREAKAKDLAYTNETGNLDTQPTL
KALVKTLLLPKDNTEHNAEATKLEALFGGLAADKTKTYLDMVDAEIIPAGIAGRTTEAPLGKIH
DTVELGDILSNYEMIAAQNVVTLKKNLDAVSKKQQTESAENKEKICNAAKDNQKACENLKEKG
CVFNTESNKCELKKDVKEKLEKESKETEGKDEKANTTGSNSFLIHKAPLLLAFLLF

SEQ ID NO: 2 >Tb427VSG-2: GB Accession X56762.1 | Trypanosoma brucei brucei |
Lister427 | variant surface glycoprotein MITat 1.2 (Lister 427-2|Identical in my assembly) |
Source=GenBank download 170507 | Protein length=476

MPSNQEARLFLAVLVLAQVLPILVDSAAEKGFKQAFWQPLCQVSEELDDQPKGALFTL
QAAASKIQKMRDAALRASIYAEINHGTNRAKAAVIVANHYAMKADSGLEALKQTLSSQEVTATA
TASYLKGRIDEYLNLLLQTKESGTSGCMMDTSGTNTVTKAGGTIGGVPCKLQLSPIQPKRPAATY
LGKAGYVGLTRQADAANNFHDNDAECRLASGHNTNGLGKSGQLSAAVTMAAGYVTVANSQTA
VTVQALDALQEASGAAHQPWIDAWKAKKALTGAETAEFRNETAGIAGKTGVTKLVEEALLKKK
DSEASEIQTELKKYFSGHENEQWTAIEKLISEQPVAQNLVGDNQPTKLGELEGNAKLTTILAYY
RMETAGKFEVLTQKHKPAESQQQAAETEGSCNKKDQNECKSPCKWHNDAENKKCTLDKEEA
KKVADETAKDGKTGNTNTTGSSNSFVISKTPLWLAVLLF

SEQ ID NO: 3 >Tb427VSG-3: GB Accession AY935575.1 | Trypanosoma brucei brucei |
Lister427 | variant surface glycoprotein MITat 1.3 (Lister 427-3|Identical in my assembly) |
Source=GenBank download 170507 | Protein length=509

MQAAALLLLVLRAITSIEAAADDVNPDDNKEDFAVLCALAALANLQTTVPSIDTSGLAAY
DNLQQLNLSLSSKEWKSLFNKAADSNGSPKQPPEGFQSDPTWRKQWPIWVTAAAALKAENKE
AAVLARAGLTNAPEELRNRARLALIPLLAQAEQIRDRLSEIQKQNEDTTPTAIAKALNKAVYGQ
DKETGAVYNSADCFSGNVADSTQNSCKAGNQASKATTVAATIVCVCHKKNGGNDAANACGRLI

NHQSDAGANLATASSDFGDIIATCAARPPKPLTAAYLDSALAAVSARIRFKNGNGYLGKFKATG
CTGSASEGLCVEYTALTAATMQNFYKIPWVKEISNVAEALKRTEKDAAESTLLSTWLKASENQG
NSVAQKLIKVGDSKAVPPAQRQTQNKPGSNCNKNLKKSECKDSDGCKWNRTEETEGDFCKPK
ETGTENPAAGTGEGAAGANTETKKCSDKKTEGDCKDGCKWDGKECKDSSILATKKFALTVVSA
AFVALLF

SEQ ID NO: 4 >Tb427VSG-13: GB Accession AY935576.1 | Trypanosoma brucei brucei
| Lister427 | variant surface glycoprotein MITat 1.13 (Lister 427-13|Not fully assembled by me) |
Source=GenBank download 170507 | Protein length=499

MQRLGTAVFFLLAFRYSTEQAVGLKEPNAPCTTACGCKSRLLKRLDLYTSKYADGINNE
RENSEAYSKLVTAALAAVPTMQRKILPLLGAAADILDICRRELATARPLVQAAISKIEEAAGVYNT
LHKLERGLGEAKIEFGGTDLRLTKTKFRATSLGTIHTADCPNADPGETNVKIGLEHEENEPEPA
KLITHGHLDATCASGVGQSSSCHTTAVEANTHLTLGLTFSGSSKDESATWNAATNNKRAIHSN
DADFLGSNATVAHEALKAIRSAGASTPCSSLITDFNAVRANPKFKLMVIKALLNKPTAEKESDAP
ADEVNNAINSAYGREGSEYNTKTWKDIGSTRIPKADPPGEKTDTIDKLSSLPQWGDAIARLLLQ
EITKQEEQSIKTSSDEATNKECDKHTAKTEGECTKLGCDYDAENKKCKPKSEKETTAAGKKDRA
AGETGCAKHGTDKDKCENDKSCKWENNACKDSSILATKKFALSMVSAAFVTLLF

SEQ ID NO: 5 >X56767.1 | Trypanosoma brucei brucei | ILTat1 | mRNA variant surface protein ILTat 1.24 | Source=GenBank download 170421 | Protein length=514

MVYRNILQLSVLKVLLIVLIVEATHFGVKYELWQPECELTAELRKTAGVAKMKVNSDLN
SFKTLELTKMKLLTFAAKFPESKEALTLRALEAALNTDLRALRDNIANGIDRAVRATAYASEAAG
ALFSGIQTLHDATDGTTYCLSASGQGSNGNAAMASQGCKPLALPELLTEDSYNTDVISDKGFPKI
SPLTNAQGQGKSGECGLFQAASGAQATNTGVQFSGGSRINLGLGAIVASAAQQPTRPDLSDFSG
TARNQADTLYGKAHASITELLQLAQGPKPGQTEVETMKLLAQKTAALDSIKFQLAASTGKKTSD
YKEDENLKTEYFGKTESNIEALWNKVKEEKVKGADPEDPSKESKISDLNTEEQLQRVLDYYAVA
TMLKLAKQAEDIAKLETEIADQRGKSPEAECNKITEEPKCSEEKICSWHKEVKAGEKNCQFNST
KASKSGVPVTQTQTAGADTTAEKCKGKGEKDCKSPDCKWEGGTCKDSSILANKQFALSVASAA
FVALLF

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Drug-decorated VSG coats

The inventors have generated tools to derivatize the dense and homogeneous surface coat of the African trypanosome (*T. brucei*) for use as a display platform for (any) antigens to which antibodies need to be raised. These tools consist of:
(a) A specific vector to efficiently replace the expressed VSG (VSG2) with any other VSG of interest (see below). **Figure 1** contains the maps of two such plasmid vectors.
(b) Specific sets of VSGs of interest: these contain extended N-termini that are accessible to the enzyme sortase (here, derived from *Streptococcus pyogenes*). N-termini accessibility is determined by (i) relative placement on the VSG (determined structurally - **Figure 2** contains VSG2 and VSG3 whose N-termini are accessible, and as a comparison also VSG13, whose N-terminus is not accessible because of steric hindrance). It is also determined by (ii) the initiating amino acids, which must be Ala-Ala for the particular sortase employed (or Gly-Gly for sortases from other organisms).
(c) VSGs that fulfil those criteria are engineered into a modified Lister 427 strain of *T. brucei* by replacing the active VSG (see (a) above - **Figure 4** contains the amino acid sequences of three such VSGs: VSG3 (A), VSG2 (B) and ILTat1.24 (C); It is worth mentioning that the VSG3 that is engineered for sortagging purposes, contains a mutation (S317A) that removes a native glycosylation event which the inventors have recently shown to be immune-suppressive - Pinger et al., Nat. Microbiology, 2018).
(d) The modification of the Lister 427 strain of T. brucei consists of genetic deletion of the endogenous glycophosphatidylinositol phospholipase C (GPI-PLC), the enzyme that "sheds" VSG off the surface of dying cells (this is crucial to generating *T. brucei* that can be used as vaccine display platform, because unless GPI-PLC is removed from the genome, any form of inactivation of the parasite (e.g. UV-irradiation), that is crucial (i) to disallow switching and loss of the engineered VSG and (ii) to remove infectivity, will also lead to the disintegration of the VSG coat and of the cell itself (once VSGs are shed due to the action of GPI-PLC, the VSG coat disintegrates and the cells lyse - **Figure 5** explains that concept).

The herein disclosed method depends on "highjacking" the natural ability of *T. brucei* to elicit a neutralizing (and long-lasting) antibody response to its VSG coat, to produce antibodies at will. The inventors do this by decorating the *T. brucei* VSG coat not only with any peptide epitope/antigen but also sugars, lipids or small molecules and then using the decorated VSG coat as a vaccine carrier. Specifically, the inventors use the enzyme sortase A to covalently ligate any moiety to VSG coats genetically engineered to carry N-terminal sortase acceptor sequences **(****Figures 4** **and** **6**).

Therefore, the inventors produce a His-tagged sortase A, derived from *Streptococcus pyogenes,* in *E.coli,* using a plasmid containing the S. pyogenes-derived sortase A expression construct (pSpSortA-pET28a). This plasmid is transformed into BL21 DE3 cells (Life Technologies C6000-03). Colonies from this transformation are used to inoculate large cultures of LB media (Sigma-Aldrich, L3022-1KG) which are then grown shaking at 37°C to an optical density (OD600) of 0.4 - 0.8. Cultures are induced with 1 mM IPTG, grown for an additional 3-4 h and harvested by centrifugation. Cell pellets are resuspended in TBS/imidazole (20 mM Tris, 150 mM NaCl, 20 mM imidazole), and lysed using an EmulsiFlex-C5 homogenizer (Avestin). DNase-A powder (Sigma D5025) and 5 mM 2-Mercaptoethanol (2-ME) are added to the lysate, which is then clarified by centrifugation to remove particulates. The supernatant is passed through a column packed with Ni-NTA agarose beads (QIAGEN, 30230) equilibrated with Wash Buffer (20 mM Tris, 300 mM NaCl, 20 mM imidazole, 5 mM 2-ME). The column is then washed with 100 ml of Wash Buffer and eluted with 30-35 ml of Elution Buffer (20 mM Tris, 300 mM NaCl, 200 mM imidazole, 5 mM 2-ME). Samples containing protein are then pooled and dialyzed in Dialysis Buffer (20 mM Tris, 150 mM NaCl, 1 mM DTT). The resulting sample is concentrated using a centrifugal filter unit (Amicon Ultra-15, 10,000 NMWL, Merck Millipore), aliquoted and stored at -80°C for future use.

The sortagging reaction is performed as follows: a mixture of sortagging solution containing 100 uM purified sortase A and 300-600 uM sortaggable-peptide in HMI-9 media is incubated on ice for 30-60 min (a sortaggable peptide includes any peptide with a C-terminal sortase donor sequence, LPSTGG, that can be attached at its N-terminus to another moiety; that moiety can be a fluorophore like 6-FAM, a small molecule like 4-Hydroxy-3-nitrophenyl acetyl (NP) hapten or other small molecules that are drugs of abuse (e.g. fentanyl *etc.*). GPI-PLC-negative *T. brucei* cells expressing engineered VSGs are then pelleted, resuspended in the sortagging mixture and incubated for 60 min at 4°C on an inversion rotator. Cells are then pelleted, washed once with HMI-9 media and pelleted again before final resuspension in HMI-9 media (Hirumi and Hirumi, J. Parasitology, 1989). The efficiency of sortagging can be determined by direct FACS analysis or fluorescence microscopy (e.g. for fluorophores like 6-FAM) or by using specific monoclonal antibodies that bind the moieties decorating the VSG (**Figure 7** contains examples for 6-FAM, NP hapten and fentanyl hapten).

In **proof of principle** experiments this approach was used to generate (a) robust (in comparison to NP-CGG in Alum adjuvant) and (b) of consistent quality antibodies against a small-molecule hapten (4-hydroxy-3-nitrophenylacetyl or NP) (**Figure 8**).

This approach can be used for a range of other small molecules (e.g. drugs of abuse like cocaine, nicotine, fentanyl, carfentanyl, tramadol, ketamine etc., but also chemotherapeutics like platinum, Adriamycin *etc.;* and also small molecules that are industrial by-products of chemical reactions), for toxins that mediate allergic reactions (e.g. aflatoxin and others) for specific peptides that function as important epitopes for infectious diseases (e.g. Plasmodium-derived peptides), for glycosylated or lipidated peptides (e.g. the aberrantly-glycosylated mucin peptides that have been considered as targets for anti-cancer vaccines etc.).

From the perspective of an anti-fentanyl (anti-overdose) vaccine, the major focus is to use this system to vaccinate "at risk" individuals (defined as individuals who are regular users or substance abusers but are not yet addicted/chemically dependent, or addicted individuals leaving rehabilitation centers, as proactive protection against overdose in case of recidivism, which typically will occur within the first two months after leaving rehab). **Proof of concept** that this has been achieved using the approach herein, is provided in **Figures 9** **and** **10****.** Additionally, when this approach is coupled to repertoire analysis (**Figure 11**), it will yield a wealth of anti-fentanyl monoclonal antibodies of varying affinities (directly accessible and ready to reconstitute from the paired immunoglobulin heavy and light chain sequences generated as a result of repertoire sequencing - **Figure 11**). Such monoclonal antibody "sponges" can be used directly for therapeutic applications (e.g. anti-fentanyl antibody infusion together with methadone maintenance to curb bioavailability as well as cravings and accelerate therapeutic outcomes; or injection in the ER to blunt the effects of overdose in conjunction with naloxone - which acts quickly by antagonizing fentanyl binding to opioid receptors but which is metabolized faster than fentanyl, allowing delayed intoxication).

**The Methodology:** The ability of trypanosomes to stimulate a robust immune response in the infected individual (a response that is both long-lasting and neutralizing) is well documented. This invention renders this possible, at least in part, due to the discovery that a trypanosome's VSG protein is tolerant to the display of exogenous moieties with high efficiency on its surface using a bacterial transpeptidase sortase-based system (henceforth "sortagging").

Specifically, a sortase acceptor sequence specific to the sortase (for sortase A derived from *Streptococcus pyogenes* that is Ala-Ala and for Sortase A derived from *Staphylococcus aureus* that is Gly-Gly) can be added at the exposed N-terminal part of the VSG protein which, when it gets transported to the surface of the trypanosome, remains accessible to the sortase (see **Figures 2** **and** **6**). It is noted that VSGs initiate with the Methionine of a signal peptide, but that peptide is cleaved upon maturation - hence the mature VSG sequence is not initiating with Methionine. For instance, both VSG2 and VSG3 (the preferred VSG variants) initiate with Ala-Ala, however the exact initiating amino acid must be empirically determined (using Edman degradation, which the inventors have done for both VSGs). Finally, while the endogenous Ala-Ala is present, it is inaccessible to sortase (and requires a short N-terminal extension as shown in **Figure 4**). A complementary sortase donor sequence is then added C-terminally to the peptide/small molecule of interest (the actual sequence also depends on the sortase used; for sortase A derived from *Streptococcus pyogenes,* it is LPXTGG). LPXTGG can be added to a small molecule or other moiety with a reactive group (here the inventors use 6-FAM, or the hapten 4-hydroxy-3-nitrophenylacetyl abbreviated as NP, or Fen- **Figure 7**).

For fentanyl, the N-terminus of the sortase sequence is linked via an amide bond to the fentanyl hapten at the position located in **Figure 7D**. While most moieties can be derivatized, a derivatization that retains the antigenicity of the small molecule-peptide conjugate (e.g. NP-GGGSLPSTGG) must be empirically determined (for example this was done through trial and error for nicotine and other small molecules, e.g. NicVax). The sortase then ligates NP-carrying peptide to the exposed N-terminus of the VSG on the surface of the trypanosomes (the inventors validate this using anti-small molecule antibodies, **Figure 7**). It is also possible to insert the sortase signal sequence within the loops of VSG (as done for FLAG peptide in Stavropoulos and Papavasiliou, 2010). However in such situations it is advisable to use the sortase donor sequence (e.g. LPXTGG) within the VSG loops and the sortase acceptor sequence (e.g. Ala-Ala) on the decorating small molecule, to increase specificity. Due to the dense coat of VSGs on the surface of trypanosomes, the small molecule is then densely displayed on the surface. Upon trypanosome injection into a mammalian host, the small molecule-conjugated VSG coat is exposed to the host immune system which mounts a similar strong and specific priming immune response against the exposed hapten (e.g. NP) as it does against the VSG in natural infection (**Figure 8**). Boosting can then be achieved with hapten-VSG conjugate either on the full coat (**Figure 8**) or formulations thereof (e.g. soluble haptenated VSG but other formulations as well) achieving a scalability that is unique to this vaccine platform.

Interestingly, primary responses elicited by different VSGs are not cross-reactive (i.e. antibodies raised to VSG2 do not cross-react with VSG3 etc. Pinger et al., Nat. Communications, 2017). This suggests that each specific VSG elicits a unique subset of B cell specificities (thus, a unique repertoire) which could be more or less potent toward a specific set of small-molecule haptens. In context of the invention, specific VSGs are selected for specific haptens, for the elicitation of optimal anti-hapten responses (with some VSGs better platforms for certain haptens - **Figure 7**). For example, good anti-HA (pan-influenza) antibodies require engagement of IGHV1-69, a "rare" IGH within the general repertoire. A VSG that elicits IGHVi-69 might therefore be engaged preferably if one desired to use this method to elicit polyspecific anti-influenza antisera (e.g. in a "pan-flu" vaccine). Direct proof that VSGs elicit distinct B cell repertoires (and that this system can provide a range of different platforms depending on the preference of B cell to be elicited) is provided in **Figure 11****.**

Biosafety concerns (e.g. disease causation) but also a need to block natural switching away from the haptenated VSG, dictate that derivatized trypanosome coats are inactivated (and thus unable to cause infection). The inventors have achieved this via UV-crosslinking of trypanosomes that lack the enzyme glycophosphatidylinositol phospholipase C (GPI-PLC) and are therefore dead - but with an intact VSG coat (trypanosomes wildtype for GPI-PLC disintegrate upon UV-inactivation as GPI-PLC cleaves the GPI linkage of each VSG off the membrane and sheds the coat (as depicted in **Figure 5**). UV-crosslinking (of GPI-PLC-negative trypanosomes is achieved by pelleting cells from culture, washing with irradiation buffer (PBS supplemented with 55 mM Glucose), and resuspending in the same buffer to a density of 10⁷ cells/ml. 1 ml of this suspension is then aliquoted into each well of a 6-well tissue culture plate (Thermoscientific, 150239). Plates are UV-irradiated for 8 cycles, each cycle 30 S using a UVP crosslinker (Analytik Jena). Plates are swirled between irradiation cycles to ensure equal irradiation of trypanosomes. Irradiated cells are then resuspended at a concentration of 15 x 10⁶ cells/ml. 200 µl of this solution (3 x 10⁶ trypanosomes) can be injected intraperitoneally or subcutaneously into mice.

Overall, using this inactivation protocol and sortaggable VSGs, the inventors have generated an optimal and flexible platform for the immunogenic display of antigenic determinants toward the generation of antibodies to small-molecule haptens and peptides, which can be expanded to a wide variety of antigenic entities (e.g. lipids, nucleic acids, etc.) Proof of concept regarding generation of antibodies to small molecules (e.g. NP) is shown in **Figure 9****.** Proof of principle that such antibodies can be raised against fentanyl and when generated protect against intoxication is provided in **Figure 9** **and** **10****.** A cartoon version of the overall method is illustrated for the small molecule 6-FAM in **Figure 6****.**

An example of how to integrate the engineered VSGs of the invention into the *T.brucei* genome is provided in **Figure 3****.**

**Generalizability of the approach:** For the purposes of this application, the inventors focus on active immunotherapy against fentanyl, an adulterant of synthetic heroin and the cause of the majority of drug overdoses in the United States. This is because the inventors have tools already available (fentanyl haptenated to LPXTGG so that it can be sortagged; anti-fentanyl antibodies to verify sortaggability - from M. Pravetoni). However it should be clear that this approach can easily be adapted to raise effective antibodies against other drugs and drug metabolites (e.g. acetaminophen metabolites which cause liver toxicity, small molecules that are the toxins causal to anaphylactic shock in certain foodstuff allergies etc.). The approach can also be used for the haptenation with peptides derived from pathogens (e.g. the NANP tandem repeat, a major antigenic determinant of the Circumsporozoite protein of *Plasmodium falciparum)* or with aberrantly-glycosylated peptides unique to cancer cells (e.g. mucin) which can be used as anti-cancer vaccines (PMID: 20403708).

### Example 2: A unique prime-boost combination for the generation of high-quality antibody responses and B cell memory

The immunization scheme developed in the experiments of figure 8 and 9 above was further tested. The present invention is based on a different way to prime and boost, that does not require adjuvant but appears to elicit cognate T-cell help to small molecules in a physiological fashion. Specifically, the inventors used a hapten-carrier combination in a prime-boost combination (e.g. fentanyl covalently attached to trypanosome VSG protein) but in two completely distinct biophysical entities: the entire VSG coat of a trypanosome (a micrometer size particle which is highly dense and repetitive) followed by the same carrier moiety but now in soluble form (Figure 12).

It was found that the haptenated VSG particle, despite its size, presents a highly immunodominant surface that can elicit a highly specific antibody repertoire and a predominantly IgM response (Figure 13A). Conversely, it was found that the same moiety but now used as the soluble form during the boost, can elicit high IgG titers (equivalent in proportion to the carrier as to the hapten and also memory (Figure 13B). Neither the priming nor the boost step requires adjuvant, suggesting that the invention is based on an, to date, unknown physiological mechanism to generate high antibody titers and memory. Furthermore, the amounts used are about 250x lower than those used in standard protocols (e.g. 100 µg NP-CGG per mouse), and lead to titers that are long lasting (e.g. still increasing even one month after the first boost) (Figure 13C).

### Example 3: Specific Priming Boosting Protocol - Dosing and Timing

Herein it was generally dosed twice within 30 days as the priming step (day 0 and day 30). This dual step is not essential but may help activate rare B-cell receptors from the germline repertoire. Then the inventors boosted at day 60 or day 60 and day 90. The day 60 boost leads to a recall IgG response that continues to increase in titers for the subsequent 30 days (Figure 13C).

As mentioned, a dose that is about 250X lower than that delivered with standard methods is sufficient in the protocol of the invention. However, dose escalation (increase) at the priming step is not necessarily beneficial and potentially even detrimental (injection of material representing more than 50 million cells results in decreased titers, compared to injection representing 3 or 10 million cells - Figure 14). An optimized protocol includes a priming dose representing about 5 million cells per bout 20 g of body weight - it resulted in an IgG response that is substantial (about 15% of total IgG in a mouse (2.38 mg/ml total IgG in a mouse (Klein-Schneegans et al., 1989))- Figure 15). Thus, a two-step prime/boost process should be used in higher organisms as well, starting with a small dose as stated above.

In the below protocol details on the successful immunization schedule, using fentanyl as an example, are provided.

Six 6-8 weeks old female C57BL/6J mice per group were immunized with 5E6 cells of UV-irradiated sortagged VSG3-fentanyl cells (VSG3-Fen) subcutaneously (s.c.) in 200 µl of PBS at days 0 and 30. As control, 6 mice were either left unimmunized or injected with non-tagged UV-irradiated VSG3 carrier.

Two boosts were done using non-adjuvanted soluble VSG3 or VSG3-Fen in PBS at days 60 and 90. Blood samples were collected from the facial vein 4 days before starting the immunizations (day -4), 2 days before the first (day 58) and 8 days after the first (day 68) and second boosts (day 88 and day 98) with soluble VSG3 or VSG3-Fen proteins. [This material can be prepared ahead of time, concentrated and frozen in PBS; it can be thawed and diluted in PBS at the proper dose, prior to injection].

Results are shown in figure 16.

Specific IgG antibody titers against both Fen-hapten and VSG3 carrier protein in sera were measured by ELISA using 96-well plates coated with BSA-Fen conjugates or purified VSG3.

**Table 1: Immunization Schedule:**

| **Group** | **1^{st} Immunization (Day 0, S.C.)** | **2^{nd} Immunization (Day 30, S.C.)** | **3^{rd} Immunization (Day 60, S.C.)** | **4^{th} Immunization (Day 90, S.C.)** |
|---|---|---|---|---|
| VSG3-Fen | VSG3-Fen cells (5E6 cells, in PBS or PBS-glucose) | VSG3-Fen cells (5E6 cells, in PBS or PBS-glucose) | 100 µg VSG3-Fen in PBS (no adjuvant) | 100 µg VSG3-Fen in PBS (no adjuvant) |
| VSG3 (carrier control) non-immunized | VSG3 cells (5E6 cells, in PBS) | VSG3 cells (5E6 cells, in PBS) | 100 µg VSG3 in PBS (no adjuvant) | 100 µg VSG3 in PBS (no adjuvant) |

## Claims

1. **A method for eliciting an immune response, and/or for amplifying a pre-existing immune response, against an antigenic compound in a subject,** comprising at least one priming-immunization step and at least one boosting-immunization step, wherein the at least one boosting-immunization step is performed after the at least one priming-immunization step, and wherein:
(i) The at least one priming-immunization step comprises an immunization of the mammal with an array of immunization-carrier-fusions, and wherein each immunization-carrier-fusion is composed of a carrier protein fused to the antigenic compound; and
(ii) The at least one boosting-immunization step comprises an immunization of the mammal with the immunization-carrier-fusion used in (i) in a non-aggregated, preferably soluble form.

2. The method of claim 1, wherein the immunization-carrier-fusion is an antigenic particle coated with an engineered variant surface glycoprotein (eVSG), wherein the eVSG comprises the antigenic compound covalently linked, optionally via a linker, to the N-terminus of the VSG carrier protein.

3. The method of claim 1 or 2, wherein the immunization-carrier-fusion has the following (covalent) structure from N- to C-terminus: antigenic compound, a sortagging donor sequence, a sortagging acceptor sequence, a linker, a VSG protein sequence.

4. The method of any one of claims 1 to 3, wherein the antigenic compound is a disease associated antigen, for example a cancer antigen, an allergen or a viral immunogen, or is a dependency causing substance selected from (i) delta-9-tetrahydrocannabinol (THC) or Synthetic cannabinoids, such as classical cannabinoids, non-classical cannabinoids, hybrid cannabinoids, aminoalkylindoles, and eicosanoids; for example Δ9-THC HU-210, (C8) CP 47,497, JWH-018, AM-2201 (Fluorinated JWH-018), UR-144, XLR-11 (Fluorinated UR-144), APICA, STS-135 (Fluorinated APICA). AB-PINACA, PB-22, 5F-PB-22 (Fluorinated PB-22); or (ii) methamphetamine and derivatives thereof such as 3,4-methylenedioxy-methamphetamine (MDMA)Ecstasy/Molly; or (iii) a synthetic cathinone like alpha-pyrrolidinopentiophenone (alpha-PVP); or (iv) an opioid including heroin, synthetic opioids such as fentanyl or related compounds such as carfentanyl, and other opioid pain relievers, such as oxycodone (OxyContin®), hydrocodone (Vicodin®), codeine, morphine, desomorphine (Krokodil); or (v) steroids (anabolic substances), or is nicotine.

5. The method of any one of claims 1 to 4, wherein the carrier protein is a VSG protein derived from *Trypanosoma brucei.*

6. The method of any one of the preceding claims, wherein the aggregate of immunization-carrier-fusions constitutes a particle coated with multiple immunization-particle fusions.

7. The method of claim 6, wherein the particle is coated with more than one immunization-particle fusions, preferably more than 10 immunization-particle fusions per particle.

8. The method of any one of the preceding claims, wherein the immunization-carrier used in the method is a VSG protein, and wherein the VSG protein used as carrier in (i) and (ii) are derived from the same VSG protein type, for example the VSG in both (i) and (ii) are VSG3 of *Trypanosoma brucei.*

9. The method of any one of claims 1 to 16, wherein the method comprises at least two priming-immunization steps, and wherein all of the priming immunization steps are performed prior to any of the boosting-immunization steps.

10. The method of any one of claims 1 to 9, wherein the method comprises at least two boosting-immunization steps, and wherein all of the boosting immunization steps are performed after any of the priming-immunization steps

11. The method of any one of the preceding claims, wherein the subject is selected from an animal having an adaptive immune system, such as vertebrates such as birds (chicken), and mammals such as mouse, rabbit, camel, goat, rat, dog, cat, monkey, hamster or other mammals, or is a human, such as a human patient in need of an elicited or amplified immune response.

12. **A non-therapeutic method for generating antibodies against an antigenic-compound,** the method comprising performing in a non-human animal the method of any one of claims 1 to 11, and isolating from the non-human animal antibodies or B-cells producing antibodies against the antigenic compound.

13. **An antibody, or an antibody producing B-cell,** generated and/or isolated according to a method of claim 12.

14. **An immunization kit,** the immunization kit comprising (a) an aggregate of immunization-carrier protein recited in any one of claims 1 to 11, and (b) a non-aggregated, preferably soluble form, of the immunization-carrier protein of (a); wherein the immunization-carrier protein of (a) and (b) are capable to be fused to an antigenic compound to obtain an immunization-carrier-fusion recited in any one of claims 1 to 11.

15. **An immunization-carrier-fusion for use in a method of treating or preventing an adverse condition in a subject by eliciting an immune response, and/or for amplifying a pre-existing immune response, against an antigenic compound which causes or is associated with the adverse condition,** the treatment comprising performing in the subject the steps of the method of any one of claims 1 to 11.


SEQUENCE LISTING

<110>  Deutsches Krebsforschungszentrum Stiftung des oeffentlichen
       Rechts

<120>  Immunization scheme for variant surface glycoprotein carriers

<130>  D31719EP

<150>  PCT/EP2019/079063
<151>  2019-10-24

<160>  17

<170>  PatentIn version 3.5

<210>  1
<211>  492
<212>  PRT
<213>  Trypanosoma brucei

<400>  1

Met Ala Thr Gly Arg Ala Lys Asn Thr Lys Trp Ala Arg Trp Leu Ser
1               5                   10                  15

Thr Ala Gly Leu Ile Ile Val Val Thr Leu Pro Ala Thr Thr Met Ala
            20                  25                  30

Ala Glu Arg Thr Gly Leu Lys Ala Thr Ala Trp Lys Pro Leu Cys Lys
        35                  40                  45

Leu Thr Thr Glu Leu Ser Lys Val Ser Gly Glu Met Leu Asn Glu Gly
    50                  55                  60

Gln Glu Val Ile Ser Asn Ile Gln Lys Ile Lys Ala Ala Glu Tyr Lys
65                  70                  75                  80

Val Ser Ile Tyr Leu Ala Lys Asn Pro Glu Thr Gln Ala Leu Gln Gln
                85                  90                  95

Leu Thr Leu Leu Arg Gly Tyr Phe Ala Arg Lys Thr Asn Gly Gly Leu
            100                 105                 110

Glu Ser Tyr Lys Thr Met Gly Leu Ala Thr Gln Ile Arg Ser Ala Arg
        115                 120                 125

Ala Ala Ala Tyr Leu Lys Gly Ser Ile Asp Glu Phe Leu Asn Leu Leu
    130                 135                 140

Glu Ser Leu Lys Gly Gly Ser Glu Asn Lys Cys Leu Val Thr Thr Asn
145                 150                 155                 160

Ala Asp Thr Ala Ala Thr Arg Arg Glu Thr Lys Leu Asp Asp Gln Glu
                165                 170                 175

Cys Ala Leu Ser Met Pro Glu Thr Lys Pro Glu Ala Ala Thr Arg Thr
            180                 185                 190

Glu Leu Thr Gln Thr Gly Tyr Pro Asn Leu Gln His Gly Gly Gly Gly
        195                 200                 205

Thr Ala Asn Thr Phe Gln Pro Thr Thr Ser Thr Gly Thr Cys Lys Leu
    210                 215                 220

Leu Ser Gly His Ser Thr Asn Gly Tyr Pro Thr Thr Ser Ala Leu Asp
225                 230                 235                 240

Thr Thr Ala Lys Val Leu Ala Gly Tyr Met Thr Ile Pro Asn Thr Gln
                245                 250                 255

Val Glu Ala Thr Leu Ala Asn Met Gln Ala Met Gly Asn Gly His Lys
            260                 265                 270

Ala Thr Ala Pro Ala Trp His Glu Ala Trp Glu Ala Arg Asn Arg Glu
        275                 280                 285

Ala Lys Ala Lys Asp Leu Ala Tyr Thr Asn Glu Thr Gly Asn Leu Asp
    290                 295                 300

Thr Gln Pro Thr Leu Lys Ala Leu Val Lys Thr Leu Leu Leu Pro Lys
305                 310                 315                 320

Asp Asn Thr Glu His Asn Ala Glu Ala Thr Lys Leu Glu Ala Leu Phe
                325                 330                 335

Gly Gly Leu Ala Ala Asp Lys Thr Lys Thr Tyr Leu Asp Met Val Asp
            340                 345                 350

Ala Glu Ile Ile Pro Ala Gly Ile Ala Gly Arg Thr Thr Glu Ala Pro
        355                 360                 365

Leu Gly Lys Ile His Asp Thr Val Glu Leu Gly Asp Ile Leu Ser Asn
    370                 375                 380

Tyr Glu Met Ile Ala Ala Gln Asn Val Val Thr Leu Lys Lys Asn Leu
385                 390                 395                 400

Asp Ala Val Ser Lys Lys Gln Gln Thr Glu Ser Ala Glu Asn Lys Glu
                405                 410                 415

Lys Ile Cys Asn Ala Ala Lys Asp Asn Gln Lys Ala Cys Glu Asn Leu
            420                 425                 430

Lys Glu Lys Gly Cys Val Phe Asn Thr Glu Ser Asn Lys Cys Glu Leu
        435                 440                 445

Lys Lys Asp Val Lys Glu Lys Leu Glu Lys Glu Ser Lys Glu Thr Glu
    450                 455                 460

Gly Lys Asp Glu Lys Ala Asn Thr Thr Gly Ser Asn Ser Phe Leu Ile
465                 470                 475                 480

His Lys Ala Pro Leu Leu Leu Ala Phe Leu Leu Phe
                485                 490

<210>  2
<211>  476
<212>  PRT
<213>  Trypanosoma brucei

<400>  2

Met Pro Ser Asn Gln Glu Ala Arg Leu Phe Leu Ala Val Leu Val Leu
1               5                   10                  15

Ala Gln Val Leu Pro Ile Leu Val Asp Ser Ala Ala Glu Lys Gly Phe
            20                  25                  30

Lys Gln Ala Phe Trp Gln Pro Leu Cys Gln Val Ser Glu Glu Leu Asp
        35                  40                  45

Asp Gln Pro Lys Gly Ala Leu Phe Thr Leu Gln Ala Ala Ala Ser Lys
    50                  55                  60

Ile Gln Lys Met Arg Asp Ala Ala Leu Arg Ala Ser Ile Tyr Ala Glu
65                  70                  75                  80

Ile Asn His Gly Thr Asn Arg Ala Lys Ala Ala Val Ile Val Ala Asn
                85                  90                  95

His Tyr Ala Met Lys Ala Asp Ser Gly Leu Glu Ala Leu Lys Gln Thr
            100                 105                 110

Leu Ser Ser Gln Glu Val Thr Ala Thr Ala Thr Ala Ser Tyr Leu Lys
        115                 120                 125

Gly Arg Ile Asp Glu Tyr Leu Asn Leu Leu Leu Gln Thr Lys Glu Ser
    130                 135                 140

Gly Thr Ser Gly Cys Met Met Asp Thr Ser Gly Thr Asn Thr Val Thr
145                 150                 155                 160

Lys Ala Gly Gly Thr Ile Gly Gly Val Pro Cys Lys Leu Gln Leu Ser
                165                 170                 175

Pro Ile Gln Pro Lys Arg Pro Ala Ala Thr Tyr Leu Gly Lys Ala Gly
            180                 185                 190

Tyr Val Gly Leu Thr Arg Gln Ala Asp Ala Ala Asn Asn Phe His Asp
        195                 200                 205

Asn Asp Ala Glu Cys Arg Leu Ala Ser Gly His Asn Thr Asn Gly Leu
    210                 215                 220

Gly Lys Ser Gly Gln Leu Ser Ala Ala Val Thr Met Ala Ala Gly Tyr
225                 230                 235                 240

Val Thr Val Ala Asn Ser Gln Thr Ala Val Thr Val Gln Ala Leu Asp
                245                 250                 255

Ala Leu Gln Glu Ala Ser Gly Ala Ala His Gln Pro Trp Ile Asp Ala
            260                 265                 270

Trp Lys Ala Lys Lys Ala Leu Thr Gly Ala Glu Thr Ala Glu Phe Arg
        275                 280                 285

Asn Glu Thr Ala Gly Ile Ala Gly Lys Thr Gly Val Thr Lys Leu Val
    290                 295                 300

Glu Glu Ala Leu Leu Lys Lys Lys Asp Ser Glu Ala Ser Glu Ile Gln
305                 310                 315                 320

Thr Glu Leu Lys Lys Tyr Phe Ser Gly His Glu Asn Glu Gln Trp Thr
                325                 330                 335

Ala Ile Glu Lys Leu Ile Ser Glu Gln Pro Val Ala Gln Asn Leu Val
            340                 345                 350

Gly Asp Asn Gln Pro Thr Lys Leu Gly Glu Leu Glu Gly Asn Ala Lys
        355                 360                 365

Leu Thr Thr Ile Leu Ala Tyr Tyr Arg Met Glu Thr Ala Gly Lys Phe
    370                 375                 380

Glu Val Leu Thr Gln Lys His Lys Pro Ala Glu Ser Gln Gln Gln Ala
385                 390                 395                 400

Ala Glu Thr Glu Gly Ser Cys Asn Lys Lys Asp Gln Asn Glu Cys Lys
                405                 410                 415

Ser Pro Cys Lys Trp His Asn Asp Ala Glu Asn Lys Lys Cys Thr Leu
            420                 425                 430

Asp Lys Glu Glu Ala Lys Lys Val Ala Asp Glu Thr Ala Lys Asp Gly
        435                 440                 445

Lys Thr Gly Asn Thr Asn Thr Thr Gly Ser Ser Asn Ser Phe Val Ile
    450                 455                 460

Ser Lys Thr Pro Leu Trp Leu Ala Val Leu Leu Phe
465                 470                 475

<210> 3
<211> 509
<212> PRT
<213> Trypanosoma brucei

<400> 3

Met Gln Ala Ala Ala Leu Leu Leu Leu Val Leu Arg Ala Ile Thr Ser
1               5                   10                  15

Ile Glu Ala Ala Ala Asp Asp Val Asn Pro Asp Asp Asn Lys Glu Asp
            20                  25                  30

Phe Ala Val Leu Cys Ala Leu Ala Ala Leu Ala Asn Leu Gln Thr Thr
        35                  40                  45

Val Pro Ser Ile Asp Thr Ser Gly Leu Ala Ala Tyr Asp Asn Leu Gln
    50                  55                  60

Gln Leu Asn Leu Ser Leu Ser Ser Lys Glu Trp Lys Ser Leu Phe Asn
65                  70                  75                  80

Lys Ala Ala Asp Ser Asn Gly Ser Pro Lys Gln Pro Pro Glu Gly Phe
                85                  90                  95

Gln Ser Asp Pro Thr Trp Arg Lys Gln Trp Pro Ile Trp Val Thr Ala
            100                 105                 110

Ala Ala Ala Leu Lys Ala Glu Asn Lys Glu Ala Ala Val Leu Ala Arg
        115                 120                 125

Ala Gly Leu Thr Asn Ala Pro Glu Glu Leu Arg Asn Arg Ala Arg Leu
    130                 135                 140

Ala Leu Ile Pro Leu Leu Ala Gln Ala Glu Gln Ile Arg Asp Arg Leu
145                 150                 155                 160

Ser Glu Ile Gln Lys Gln Asn Glu Asp Thr Thr Pro Thr Ala Ile Ala
                165                 170                 175

Lys Ala Leu Asn Lys Ala Val Tyr Gly Gln Asp Lys Glu Thr Gly Ala
            180                 185                 190

Val Tyr Asn Ser Ala Asp Cys Phe Ser Gly Asn Val Ala Asp Ser Thr
        195                 200                 205

Gln Asn Ser Cys Lys Ala Gly Asn Gln Ala Ser Lys Ala Thr Thr Val
    210                 215                 220

Ala Ala Thr Ile Val Cys Val Cys His Lys Lys Asn Gly Gly Asn Asp
225                 230                 235                 240

Ala Ala Asn Ala Cys Gly Arg Leu Ile Asn His Gln Ser Asp Ala Gly
                245                 250                 255

Ala Asn Leu Ala Thr Ala Ser Ser Asp Phe Gly Asp Ile Ile Ala Thr
            260                 265                 270

Cys Ala Ala Arg Pro Pro Lys Pro Leu Thr Ala Ala Tyr Leu Asp Ser
        275                 280                 285

Ala Leu Ala Ala Val Ser Ala Arg Ile Arg Phe Lys Asn Gly Asn Gly
    290                 295                 300

Tyr Leu Gly Lys Phe Lys Ala Thr Gly Cys Thr Gly Ser Ala Ser Glu
305                 310                 315                 320

Gly Leu Cys Val Glu Tyr Thr Ala Leu Thr Ala Ala Thr Met Gln Asn
                325                 330                 335

Phe Tyr Lys Ile Pro Trp Val Lys Glu Ile Ser Asn Val Ala Glu Ala
            340                 345                 350

Leu Lys Arg Thr Glu Lys Asp Ala Ala Glu Ser Thr Leu Leu Ser Thr
        355                 360                 365

Trp Leu Lys Ala Ser Glu Asn Gln Gly Asn Ser Val Ala Gln Lys Leu
    370                 375                 380

Ile Lys Val Gly Asp Ser Lys Ala Val Pro Pro Ala Gln Arg Gln Thr

385                 390                 395                 400

Gln Asn Lys Pro Gly Ser Asn Cys Asn Lys Asn Leu Lys Lys Ser Glu
                405                 410                 415

Cys Lys Asp Ser Asp Gly Cys Lys Trp Asn Arg Thr Glu Glu Thr Glu
            420                 425                 430

Gly Asp Phe Cys Lys Pro Lys Glu Thr Gly Thr Glu Asn Pro Ala Ala
        435                 440                 445

Gly Thr Gly Glu Gly Ala Ala Gly Ala Asn Thr Glu Thr Lys Lys Cys
    450                 455                 460

Ser Asp Lys Lys Thr Glu Gly Asp Cys Lys Asp Gly Cys Lys Trp Asp
465                 470                 475                 480

Gly Lys Glu Cys Lys Asp Ser Ser Ile Leu Ala Thr Lys Lys Phe Ala
                485                 490                 495

Leu Thr Val Val Ser Ala Ala Phe Val Ala Leu Leu Phe
            500                 505

<210>  4
<211>  499
<212>  PRT
<213>  Trypanosoma brucei

<400>  4

Met Gln Arg Leu Gly Thr Ala Val Phe Phe Leu Leu Ala Phe Arg Tyr
1               5                   10                  15

Ser Thr Glu Gln Ala Val Gly Leu Lys Glu Pro Asn Ala Pro Cys Thr
            20                  25                  30

Thr Ala Cys Gly Cys Lys Ser Arg Leu Leu Lys Arg Leu Asp Leu Tyr
        35                  40                  45

Thr Ser Lys Tyr Ala Asp Gly Ile Asn Asn Glu Arg Glu Asn Ser Glu
    50                  55                  60

Ala Tyr Ser Lys Leu Val Thr Ala Ala Leu Ala Ala Val Pro Thr Met
65                  70                  75                  80

Gln Arg Lys Ile Leu Pro Leu Leu Gly Ala Ala Ala Asp Ile Leu Asp
                85                  90                  95

Ile Cys Arg Arg Glu Leu Ala Thr Ala Arg Pro Leu Val Gln Ala Ala

            100                 105                 110

Ile Ser Lys Ile Glu Glu Ala Ala Gly Val Tyr Asn Thr Leu His Lys
        115                 120                 125

Leu Glu Arg Gly Leu Gly Glu Ala Lys Ile Glu Phe Gly Gly Thr Asp
    130                 135                 140

Leu Arg Leu Thr Lys Thr Lys Phe Arg Ala Thr Ser Leu Gly Thr Ile
145                 150                 155                 160

His Thr Ala Asp Cys Pro Asn Ala Asp Pro Gly Glu Thr Asn Val Lys
                165                 170                 175

Ile Gly Leu Glu His Glu Glu Asn Glu Pro Glu Pro Ala Lys Leu Ile
            180                 185                 190

Thr His Gly His Leu Asp Ala Thr Cys Ala Ser Gly Val Gly Gln Ser
        195                 200                 205

Ser Ser Cys His Thr Thr Ala Val Glu Ala Asn Thr His Leu Thr Leu
    210                 215                 220

Gly Leu Thr Phe Ser Gly Ser Ser Lys Asp Glu Ser Ala Thr Trp Asn
225                 230                 235                 240

Ala Ala Thr Asn Asn Lys Arg Ala Ile His Ser Asn Asp Ala Asp Phe
                245                 250                 255

Leu Gly Ser Asn Ala Thr Val Ala His Glu Ala Leu Lys Ala Ile Arg
            260                 265                 270

Ser Ala Gly Ala Ser Thr Pro Cys Ser Ser Leu Ile Thr Asp Phe Asn
        275                 280                 285

Ala Val Arg Ala Asn Pro Lys Phe Lys Leu Met Val Ile Lys Ala Leu
    290                 295                 300

Leu Asn Lys Pro Thr Ala Glu Lys Glu Ser Asp Ala Pro Ala Asp Glu
305                 310                 315                 320

Val Asn Asn Ala Ile Asn Ser Ala Tyr Gly Arg Glu Gly Ser Glu Tyr
                325                 330                 335

Asn Thr Lys Thr Trp Lys Asp Ile Gly Ser Thr Arg Ile Pro Lys Ala
            340                 345                 350

Asp Pro Pro Gly Glu Lys Thr Asp Thr Ile Asp Lys Leu Ser Ser Leu
        355                 360                 365

Pro Gln Trp Gly Asp Ala Ile Ala Arg Leu Leu Leu Gln Glu Ile Thr
    370                 375                 380

Lys Gln Glu Glu Gln Ser Ile Lys Thr Ser Ser Asp Glu Ala Thr Asn
385                 390                 395                 400

Lys Glu Cys Asp Lys His Thr Ala Lys Thr Glu Gly Glu Cys Thr Lys
                405                 410                 415

Leu Gly Cys Asp Tyr Asp Ala Glu Asn Lys Lys Cys Lys Pro Lys Ser
            420                 425                 430

Glu Lys Glu Thr Thr Ala Ala Gly Lys Lys Asp Arg Ala Ala Gly Glu
        435                 440                 445

Thr Gly Cys Ala Lys His Gly Thr Asp Lys Asp Lys Cys Glu Asn Asp
    450                 455                 460

Lys Ser Cys Lys Trp Glu Asn Asn Ala Cys Lys Asp Ser Ser Ile Leu
465                 470                 475                 480

Ala Thr Lys Lys Phe Ala Leu Ser Met Val Ser Ala Ala Phe Val Thr
                485                 490                 495

Leu Leu Phe

<210> 5
<211> 514
<212> PRT
<213> Trypanosoma brucei

<400> 5

Met Val Tyr Arg Asn Ile Leu Gln Leu Ser Val Leu Lys Val Leu Leu
1               5                   10                  15

Ile Val Leu Ile Val Glu Ala Thr His Phe Gly Val Lys Tyr Glu Leu
            20                  25                  30

Trp Gln Pro Glu Cys Glu Leu Thr Ala Glu Leu Arg Lys Thr Ala Gly
        35                  40                  45

Val Ala Lys Met Lys Val Asn Ser Asp Leu Asn Ser Phe Lys Thr Leu
    50                  55                  60

Glu Leu Thr Lys Met Lys Leu Leu Thr Phe Ala Ala Lys Phe Pro Glu
65                  70                  75                  80

Ser Lys Glu Ala Leu Thr Leu Arg Ala Leu Glu Ala Ala Leu Asn Thr
                85                  90                  95

Asp Leu Arg Ala Leu Arg Asp Asn Ile Ala Asn Gly Ile Asp Arg Ala
            100                 105                 110

Val Arg Ala Thr Ala Tyr Ala Ser Glu Ala Ala Gly Ala Leu Phe Ser
        115                 120                 125

Gly Ile Gln Thr Leu His Asp Ala Thr Asp Gly Thr Thr Tyr Cys Leu
    130                 135                 140

Ser Ala Ser Gly Gln Gly Ser Asn Gly Asn Ala Ala Met Ala Ser Gln
145                 150                 155                 160

Gly Cys Lys Pro Leu Ala Leu Pro Glu Leu Leu Thr Glu Asp Ser Tyr
                165                 170                 175

Asn Thr Asp Val Ile Ser Asp Lys Gly Phe Pro Lys Ile Ser Pro Leu
            180                 185                 190

Thr Asn Ala Gln Gly Gln Gly Lys Ser Gly Glu Cys Gly Leu Phe Gln
        195                 200                 205

Ala Ala Ser Gly Ala Gln Ala Thr Asn Thr Gly Val Gln Phe Ser Gly
    210                 215                 220

Gly Ser Arg Ile Asn Leu Gly Leu Gly Ala Ile Val Ala Ser Ala Ala
225                 230                 235                 240

Gln Gln Pro Thr Arg Pro Asp Leu Ser Asp Phe Ser Gly Thr Ala Arg
                245                 250                 255

Asn Gln Ala Asp Thr Leu Tyr Gly Lys Ala His Ala Ser Ile Thr Glu
            260                 265                 270

Leu Leu Gln Leu Ala Gln Gly Pro Lys Pro Gly Gln Thr Glu Val Glu
        275                 280                 285

Thr Met Lys Leu Leu Ala Gln Lys Thr Ala Ala Leu Asp Ser Ile Lys
    290                 295                 300

Phe Gln Leu Ala Ala Ser Thr Gly Lys Lys Thr Ser Asp Tyr Lys Glu
305                 310                 315                 320

Asp Glu Asn Leu Lys Thr Glu Tyr Phe Gly Lys Thr Glu Ser Asn Ile
                325                 330                 335

Glu Ala Leu Trp Asn Lys Val Lys Glu Glu Lys Val Lys Gly Ala Asp
            340                 345                 350

Pro Glu Asp Pro Ser Lys Glu Ser Lys Ile Ser Asp Leu Asn Thr Glu
        355                 360                 365

Glu Gln Leu Gln Arg Val Leu Asp Tyr Tyr Ala Val Ala Thr Met Leu
    370                 375                 380

Lys Leu Ala Lys Gln Ala Glu Asp Ile Ala Lys Leu Glu Thr Glu Ile
385                 390                 395                 400

Ala Asp Gln Arg Gly Lys Ser Pro Glu Ala Glu Cys Asn Lys Ile Thr
                405                 410                 415

Glu Glu Pro Lys Cys Ser Glu Glu Lys Ile Cys Ser Trp His Lys Glu
            420                 425                 430

Val Lys Ala Gly Glu Lys Asn Cys Gln Phe Asn Ser Thr Lys Ala Ser
        435                 440                 445

Lys Ser Gly Val Pro Val Thr Gln Thr Gln Thr Ala Gly Ala Asp Thr
    450                 455                 460

Thr Ala Glu Lys Cys Lys Gly Lys Gly Glu Lys Asp Cys Lys Ser Pro
465                 470                 475                 480

Asp Cys Lys Trp Glu Gly Gly Thr Cys Lys Asp Ser Ser Ile Leu Ala
                485                 490                 495

Asn Lys Gln Phe Ala Leu Ser Val Ala Ser Ala Ala Phe Val Ala Leu
            500                 505                 510

Leu Phe

<210>  6
<211>  7204
<212>  DNA
<213>  artificial

<220>
<223>  pHH-VSG3-G4S-Hyg plasmid DNA sequence

<400>  6
tcgcgcgttt cggtgatgac ggtgaaaacc tctgacacat gcagctcccg gagacggtca        60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cgagctcggt acccggggat 420

ccgatatcga aggcagcgga aagtgtgcca atgcatttta aatgacagat ttattttatt 480

aaaaatgaga acacttagaa tatttcatgt cgattcaagc tagcagtaat atgccagtaa 540

atagtttcca tttacaagca caatttcatc ctccttacgt gattctgtag catttagttc 600

aagaaaaagc aataaaagaa ttcggagacg aagagccggt tagattgcat ttggacaccg 660

ctatacatat gttaagacac acaagcattc tatacgtaaa agatctagta tataggagca 720

acgctctgcc aaaacataat ggcaagacaa acggccgtgt ttgccgctga tgctacagaa 780

ccagcttaat ttccagaaga cgaaaatttg catgttttcc cacaatattt taattactct 840

tgaagattgt agttattcct acgcgacacg tacgcggcat gcaagcggca gcactgcttt 900

tattagtttt gcgcgcaata accagcatcg aagctgcagc cggtggcggt ggctcaggtg 960

gtggcggttc aggcggtggt ggctcagatg acgtcaatcc agatgacaac aaggaagact 1020

ttgcagtctt gtgcgcacta gctgcgctgg ccaacctcca gaccacggtg ccctcaatag 1080

acacgtcagg acttgcagcc tacgacaact tgcaacagct caacctaagc ctaagcagca 1140

aagaatggaa aagcctgttc aacaaagcgg ctgactcaaa cggatctccc aagcagccgc 1200

cggaaggatt tcaatcggac cctacttggc ggaagcagtg gcctatatgg gtaacagcag 1260

cagcagcatt aaaggccgaa aacaaagagg cagctgtcct agcgagggcg ggactaacaa 1320

acgcgccaga ggaactcaga aacagggccc ggctggcgct aataccctta ttagcccaag 1380

ccgagcaaat ccgggaccgg ctcagtgaaa tacaaaaaca aaacgaagac acgacaccaa 1440

cggcaatagc gaaggcactt aataaagccg tctacggcca ggacaaagaa acgggcgcgg 1500

tgtacaattc agcggattgc ttcagcggta acgttgcaga ctcaacccaa aactcctgca 1560

aagccgggaa ccaagcctcc aaagcgacga cagtagccgc aacgatagtt tgtgtttgcc 1620

acaaaaaaaa cggcggcaac gacgccgcaa acgcctgcgg tagactgatt aatcaccaat 1680

ccgacgctgg tgccaaccta gccaccgcca gctcagactt cggcgacata attgctacat 1740

gcgcagctcg cccgccaaaa ccattgaccg ctgcctatct agacagcgca ctagccgcgg 1800

tgagcgcgag gataaggttc aaaaacggca acggttacct gggcaaattc aaagcgacag 1860

gctgcacagg cgccgcaagt gaaggcttat gtgtcgaata cactgcccta acagcggcaa 1920

cgatgcaaaa tttttacaaa atcccgtggg taaaggagat ctcaaacgta gcggaagccc 1980
taaagaggac agaaaaagac gcagcagaat caacactgtt aagcacttgg cttaaagcca 2040
gcgaaaacca aggaaatagc gtcgctcaga agcttataaa ggtaggagac agcaaagcgg 2100
taccaccggc acagcgacag acacaaaata agccaggatc aaactgcaat aagaacctta 2160
aaaaaagcga atgcaaagac agtgatggtt gcaaatggaa caggactgag gagaccgaag 2220
gtgatttctg caaacctaaa gagacaggaa cagaaaaccc agcagcagga acaggagagg 2280
gagctgcagg agcaaatacg gaaaccaaaa agtgctcaga taagaaaact gaaggcgact 2340
gcaaagatgg atgcaaatgg gatggaaaag aatgcaaaga ttcctctatt ctagcaacca 2400
agaaattcgc cctcaccgtg gtttctgctg catttgtggc cttgcttttt taatttcccc 2460
cctcaaattt cccccctcct tttaaaattt tccttgctac ttgaaaactt tttgatatat 2520
tttaacacca aaaccagccg agattttgtg ttctgtgttt tgtaagttga ctgtctgatt 2580
gtctagaaat attttctggc aactaaaatt tttttctttt ttcctgtttt ttttgtaggt 2640
aggtaggaat gggggggggg gggtagttag gtaggttagt taggttagtt agggggttag 2700
ttaggggggt taggcttagg attaggcaca gcaaggtctt ctgaaattca tgtttttttt 2760
ttttttactc tgcattgcag tctccgctct tatttagttt tgctttacgt aaggtctcgt 2820
tgctgccata aaataagcta ctagtagctt accatgaaaa agcctgaact caccgcgacg 2880
tctgtcgaga agtttctgat cgaaaagttc gacagcgtct ccgacctgat gcagctctcg 2940
gagggcgaag aatctcgtgc tttcagcttc gatgtaggag ggcgtggata tgtcctgcgg 3000
gtaaatagct gcgccgatgg tttctacaaa gatcgttatg tttatcggca ctttgcatcg 3060
gccgcgctcc cgattccgga agtgcttgac attggggaat tcagcgagag cctgacctat 3120
tgcatctccc gccgtgcaca gggtgtcacg ttgcaagacc tgcctgaaac cgaactgccc 3180
gctgttctgc agccggtcgc ggaggccatg gatgcgatcg ctgcggccga tcttagccag 3240
acgagcgggt tcggcccatt cggaccgcaa ggaatcggtc aatacactac atggcgtgat 3300
ttcatatgcg cgattgctga tccccatgtg tatcactggc aaactgtgat ggacgacacc 3360
gtcagtgcgt ccgtcgcgca ggctctcgat gagctgatgc tttgggccga ggactgcccc 3420
gaagtccggc acctcgtgca cgcggatttc ggctccaaca atgtcctgac ggacaatggc 3480
cgcataacag cggtcattga ctggagcgag gcgatgttcg gggattccca atacgaggtc 3540
gccaacatct tcttctggag gccgtggttg gcttgtatgg agcagcagac gcgctacttc 3600
gagcggaggc atccggagct tgcaggatcg ccgcggctcc gggcgtatat gctccgcatt 3660
ggtcttgacc aactctatca gagcttggtt gacggcaatt tcgatgatgc agcttgggcg 3720
cagggtcgat gcgacgcaat cgtccgatcc ggagccggga ctgtcgggcg tacacaaatc 3780
gcccgcagaa gcgcggccgt ctggaccgat ggctgtgtag aagtactcgc cgatagtgga 3840

aaccgacgcc ccagcactcg tccgagggca aaggaatagg gatcgatcct gcccatttag 3900

ttagttggct tttcccttgt ctcgtgtctt ttccgtggaa aggttcccgg agtaatctga 3960

tggcacagca gggaggtgcg cctgcaggtt ggttaggaag gggggatgat gtaaaagaag 4020

aaaatggggg gatattagac ttaggcttag gattaggatt aggattagga ttagggttaa 4080

ttttttcctc ttttttttta actcacacct ctatcctgga tttttaattt ttttttttag 4140

ccattcgcgg ctcctttttt tttttttgcg ccaatgttta atttttattt gtgttttcaa 4200

tttttttgtc aaccatgcag cggctgtttt gttatgcgga ccctaaccct cctccccccc 4260

ccccgcccgc gcacctccat ttttaaaaat ttttttaccg cgtccttcaa ccagaatttt 4320

tttaaatttt ttaatttttt ttattttccg tggttttgaa tcttaatttt tcgacggcat 4380

gcccgctact cttttttggc tttttgtttt ttcgtttttt tttgacgacg ccttttttta 4440

aatttctttt cctcgatttt tttcgttcat tttttttggt ttagtattca ttttttgaac 4500

tttagttttg catttaaatt tttaacgggt ttttgcttac attttttttt tacatcctct 4560

ttttcttttt gctttttagt tttcgacatt tttcagattt ttttcttttt tgaatttttt 4620

ttttgttaca accaggcatc gttttttttg gcggcgcccc tttttggtaa caccggcggc 4680

cacggtgttt cggattaagg ccgcgggaat tcgattaggg ttagggttag ggttagggtt 4740

agggttaggg ttagggttag ggttagggtt agggttaggg ttagggttag ggttagggtt 4800

agggttaggg ttagggttag ggttagggtt agggttaggg ttagggttag ggttagggtt 4860

agggttaggg ttagggttag ggttagggtt agggttaggg ttagggttag ggttaatcac 4920

tagctagtgg atccgatatc tctagagtcg acctgcaggc atgcaagctt ggcgtaatca 4980

tggtcatagc tgtttcctgt gtgaaattgt tatccgctca caattccaca caacatacga 5040

gccggaagca taaagtgtaa agcctggggt gcctaatgag tgagctaact cacattaatt 5100

gcgttgcgct cactgcccgc tttccagtcg ggaaacctgt cgtgccagct gcattaatga 5160

atcggccaac gcgcggggag aggcggtttg cgtattgggc gctcttccgc ttcctcgctc 5220

actgactcgc tgcgctcggt cgttcggctg cggcgagcgg tatcagctca ctcaaaggcg 5280

gtaatacggt tatccacaga atcaggggat aacgcaggaa agaacatgtg agcaaaaggc 5340

cagcaaaagg ccaggaaccg taaaaaggcc gcgttgctgg cgtttttcca taggctccgc 5400

ccccctgacg agcatcacaa aaatcgacgc tcaagtcaga ggtggcgaaa cccgacagga 5460

ctataaagat accaggcgtt tccccctgga agctccctcg tgcgctctcc tgttccgacc 5520

ctgccgctta ccggatacct gtccgccttt ctcccttcgg gaagcgtggc gctttctcat 5580

agctcacgct gtaggtatct cagttcggtg taggtcgttc gctccaagct gggctgtgtg 5640

cacgaacccc ccgttcagcc cgaccgctgc gccttatccg gtaactatcg tcttgagtcc 5700

aacccggtaa gacacgactt atcgccactg gcagcagcca ctggtaacag gattagcaga 5760

gcgaggtatg taggcggtgc tacagagttc ttgaagtggt ggcctaacta cggctacact 5820

agaagaacag tatttggtat ctgcgctctg ctgaagccag ttaccttcgg aaaaagagtt 5880

ggtagctctt gatccggcaa acaaaccacc gctggtagcg gtggtttttt tgtttgcaag 5940

cagcagatta cgcgcagaaa aaaaggatct caagaagatc ctttgatctt ttctacgggg 6000

tctgacgctc agtggaacga aaactcacgt taagggattt tggtcatgag attatcaaaa 6060

aggatcttca cctagatcct tttaaattaa aaatgaagtt ttaaatcaat ctaaagtata 6120

tatgagtaaa cttggtctga cagttaccaa tgcttaatca gtgaggcacc tatctcagcg 6180

atctgtctat ttcgttcatc catagttgcc tgactccccg tcgtgtagat aactacgata 6240

cgggagggct taccatctgg ccccagtgct gcaatgatac cgcgagaccc acgctcaccg 6300

gctccagatt tatcagcaat aaaccagcca gccggaaggg ccgagcgcag aagtggtcct 6360

gcaactttat ccgcctccat ccagtctatt aattgttgcc gggaagctag agtaagtagt 6420

tcgccagtta atagtttgcg caacgttgtt gccattgcta caggcatcgt ggtgtcacgc 6480

tcgtcgtttg gtatggcttc attcagctcc ggttcccaac gatcaaggcg agttacatga 6540

tcccccatgt tgtgcaaaaa agcggttagc tccttcggtc ctccgatcgt tgtcagaagt 6600

aagttggccg cagtgttatc actcatggtt atggcagcac tgcataattc tcttactgtc 6660

atgccatccg taagatgctt ttctgtgact ggtgagtact caaccaagtc attctgagaa 6720

tagtgtatgc ggcgaccgag ttgctcttgc ccggcgtcaa tacgggataa taccgcgcca 6780

catagcagaa ctttaaaagt gctcatcatt ggaaaacgtt cttcggggcg aaaactctca 6840

aggatcttac cgctgttgag atccagttcg atgtaaccca ctcgtgcacc caactgatct 6900

tcagcatctt ttactttcac cagcgtttct gggtgagcaa aaacaggaag gcaaaatgcc 6960

gcaaaaaagg gaataagggc gacacggaaa tgttgaatac tcatactctt cctttttcaa 7020

tattattgaa gcatttatca gggttattgt ctcatgagcg gatacatatt tgaatgtatt 7080

tagaaaaata aacaaatagg ggttccgcgc acatttcccc gaaaagtgcc acctgacgtc 7140

taagaaacca ttattatcat gacattaacc tataaaaata ggcgtatcac gaggcccttt 7200

cgtc 7204

<210> 7
<211> 7219
<212> DNA
<213> Artificial

<220>
<223> pHH-ILTat1.24-G4S-Hyg plasmid DNA sequence

<400> 7
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cgagctcggt acccggggat 420

ccgatatcga aggcagcgga aagtgtgcca atgcatttta aatgacagat ttattttatt 480

aaaaatgaga acacttagaa tatttcatgt cgattcaagc tagcagtaat atgccagtaa 540

atagtttcca tttacaagca caatttcatc ctccttacgt gattctgtag catttagttc 600

aagaaaaagc aataaaagaa ttcggagacg aagagccggt tagattgcat ttggacaccg 660

ctatacatat gttaagacac acaagcattc tatacgtaaa agatctagta tataggagca 720

acgctctgcc aaaacataat ggcaagacaa acggccgtgt ttgccgctga tgctacagaa 780

ccagcttaat ttccagaaga cgaaaatttg catgttttcc cacaatattt taattactct 840

tgaagattgt agttattcct acgcgacacg tacgcggcat ggtatacaga aatatactgc 900

aattaagcgt cctaaaagta ctacttatag tgcttatagt ggaagcaggt ggcggtggct 960

caggtggtgg cggttcaggc ggtggtggct caacgcactt cggtgtaaaa tacgagctct 1020

ggcagccaga atgcgaactg acagcggaat tgcggaaaac tgcaggggtg gcaaaaatga 1080

aagttaatag cgatttgaac tcctttaaaa cgcttgaact cacaaagatg aaattgctaa 1140

ccttcgccgc aaaatttccc gaaagcaaag aggcactaac gttacgcgct ctagaagcgg 1200

cactaaacac tgatctacga gcactacgag ataatatagc aaatggcatc gacagggctg 1260

tccgggcaac agcgtacgca tcagaggcgg caggcgcttt attttctggc atacagacgc 1320

tccatgacgc caccgacggc acgacctatt gccttagcgc aagcgggcaa ggatccaacg 1380

gcaacgctgc aatggcatca caggctgcaa aaccactagc gttaccagaa cttctaacag 1440

aagactcata caacaccgac gttatatcgg acaaagggtt cccgaagatt tcgccactaa 1500

caaatgccca aggacagggc aaaagcggcg aatgcggcct tttcaagccg gcaagcggcg 1560

ctcaggcgac aaacacaggt gtgcagttct cagggggcag caggataaac ttaggccttg 1620

gcgccatagt agcaagcgca gcccagcagc cgacacgccc ggacctaagt gatttttccg 1680

gcacagcacg aaaccaagca gatacgctct acggcaaagc acatgcttcc atcacagagt 1740

tactgcagct cgcacagggg ccgaaaccag gacagaccga agtagaaaca atgaagcttc 1800

tagcacaaaa gacagcggca ttggacagca tcaagttcca actagcagca agcacaggaa 1860

agaaaacatc agactacaaa gaagacgaaa acttgaaaac ggaatacttt ggaaagacag 1920

aaagcaatat agaagcactt tggaacaaag taaaggaaga gaaagtgaaa ggagccgacc 1980

cggaggaccc aagcaaggag tccaagatta gtgacctcaa caccgaagag cagcttcaga 2040

gagttttaga ttactacgca gtggctacaa tgttaaagtt agctaaacaa gcggaggata 2100

ttgcaaaact cgaaactgaa atagcggatc aaagaggcaa atccccagaa gccgaatgca 2160

ataaaataac cgaggaaccc aaatgcagcg aggaaaagat ttgcagttgg cataaggagg 2220

ttaaagcggg agaaaagaac tgccaattta actcaacaaa agcctcaaaa agtggtgtgc 2280

ctgtaacaca aactcaaact gcaggagccg acacgacagc agaaaagtgc aaaggcaaag 2340

gagagaaaga ttgcaaatct ccggattgca aatgggaggg cggaacttgc aaagattcct 2400

ctattctagc aaacaaacaa tttgccctca gcgtggcttc tgccgcattt gtggccttgc 2460

ttttctaatt tcccccctca aatttccccc ctccttttaa aattttcctt gctacttgaa 2520

aactttttga tatattttaa caccaaaacc agccgagatt ttgtgttctg tgttttgtaa 2580

gttgactgtc tgattgtcta gaaatatttt ctggcaacta aaattttttt cttttttcct 2640

gttttttttg taggtaggta ggaatggggg ggggggggta gttaggtagg ttagttaggt 2700

tagttagggg gttagttagg ggggttaggc ttaggattag gcacagcaag gtcttctgaa 2760

attcatgttt tttttttttt tactctgcat tgcagtctcc gctcttattt agttttgctt 2820

tacgtaaggt ctcgttgctg ccataaaata agctactagt agcttaccat gaaaaagcct 2880

gaactcaccg cgacgtctgt cgagaagttt ctgatcgaaa agttcgacag cgtctccgac 2940

ctgatgcagc tctcggaggg cgaagaatct cgtgctttca gcttcgatgt aggagggcgt 3000

ggatatgtcc tgcgggtaaa tagctgcgcc gatggtttct acaaagatcg ttatgtttat 3060

cggcactttg catcggccgc gctcccgatt ccggaagtgc ttgacattgg ggaattcagc 3120

gagagcctga cctattgcat ctcccgccgt gcacagggtg tcacgttgca agacctgcct 3180

gaaaccgaac tgcccgctgt tctgcagccg gtcgcggagg ccatggatgc gatcgctgcg 3240

gccgatctta gccagacgag cgggttcggc ccattcggac cgcaaggaat cggtcaatac 3300

actacatggc gtgatttcat atgcgcgatt gctgatcccc atgtgtatca ctggcaaact 3360

gtgatggacg acaccgtcag tgcgtccgtc gcgcaggctc tcgatgagct gatgctttgg 3420

gccgaggact gccccgaagt ccggcacctc gtgcacgcgg atttcggctc caacaatgtc 3480

ctgacggaca atggccgcat aacagcggtc attgactgga gcgaggcgat gttcggggat 3540

tcccaatacg aggtcgccaa catcttcttc tggaggccgt ggttggcttg tatggagcag 3600

cagacgcgct acttcgagcg gaggcatccg gagcttgcag gatcgccgcg gctccgggcg 3660

tatatgctcc gcattggtct tgaccaactc tatcagagct tggttgacgg caatttcgat 3720

gatgcagctt gggcgcaggg tcgatgcgac gcaatcgtcc gatccggagc cgggactgtc 3780

gggcgtacac aaatcgcccg cagaagcgcg gccgtctgga ccgatggctg tgtagaagta 3840

ctcgccgata gtggaaaccg acgccccagc actcgtccga gggcaaagga atagggatcg 3900

atcctgccca tttagttagt tggcttttcc cttgtctcgt gtcttttccg tggaaaggtt 3960

cccggagtaa tctgatggca cagcagggag gtgcgcctgc aggttggtta ggaagggggg 4020

atgatgtaaa agaagaaaat ggggggatat tagacttagg cttaggatta ggattaggat 4080

taggattagg gttaattttt tcctcttttt ttttaactca cacctctatc ctggattttt 4140

aatttttttt tttagccatt cgcggctcct tttttttttt ttgcgccaat gtttaatttt 4200

ttattgtgtt ttcaattttt ttgtcaacca tgcagcggct gttttgttat gcggacccta 4260

accctcctcc cccccccccg cccgcgcacc tccattttta aaaatttttt taccgcgtcc 4320

ttcaaccaga atttttttaa attttttaat tttttttatt ttccgtggtt ttgaatctta 4380

atttttcgac ggcatgcccg ctactctttt ttggcttttt gttttttcgt ttttttttga 4440

cgacgccttt ttttaaattt cttttcctcg atttttttcg ttcatttttt ttggtttagt 4500

attcattttt tgaactttag ttttgcattt aaatttttaa cgggtttttg cttacatttt 4560

ttttttacat cctctttttc tttttgcttt ttagttttcg acatttttca gatttttttc 4620

ttttttgaat tttttttttg ttacaaccag gcatcgtttt ttttggcggc gccccttttt 4680

ggtaacaccg gcggccacgg tgtttcggat taaggccgcg ggaattcgat tagggttagg 4740

gttagggtta gggttagggt tagggttagg gttagggtta gggttagggt tagggttagg 4800

gttagggtta gggttagggt tagggttagg gttagggtta gggttagggt tagggttagg 4860

gttagggtta gggttagggt tagggttagg gttagggtta gggttagggt tagggttagg 4920

gttagggtta atcactagct agtggatccg atatctctag agtcgacctg caggcatgca 4980

agcttggcgt aatcatggtc atagctgttt cctgtgtgaa attgttatcc gctcacaatt 5040

ccacacaaca tacgagccgg aagcataaag tgtaaagcct ggggtgccta atgagtgagc 5100

taactcacat taattgcgtt gcgctcactg cccgctttcc agtcgggaaa cctgtcgtgc 5160

cagctgcatt aatgaatcgg ccaacgcgcg gggagaggcg gtttgcgtat tgggcgctct 5220

tccgcttcct cgctcactga ctcgctgcgc tcggtcgttc ggctgcggcg agcggtatca 5280

gctcactcaa aggcggtaat acggttatcc acagaatcag gggataacgc aggaaagaac 5340

atgtgagcaa aaggccagca aaaggccagg aaccgtaaaa aggccgcgtt gctggcgttt 5400

ttccataggc tccgcccccc tgacgagcat cacaaaaatc gacgctcaag tcagaggtgg 5460

cgaaacccga caggactata aagataccag gcgtttcccc ctggaagctc cctcgtgcgc 5520

tctcctgttc cgaccctgcc gcttaccgga tacctgtccg cctttctccc ttcgggaagc 5580

gtggcgcttt ctcatagctc acgctgtagg tatctcagtt cggtgtaggt cgttcgctcc 5640

aagctgggct gtgtgcacga accccccgtt cagcccgacc gctgcgcctt atccggtaac 5700

tatcgtcttg agtccaaccc ggtaagacac gacttatcgc cactggcagc agccactggt 5760

aacaggatta gcagagcgag gtatgtaggc ggtgctacag agttcttgaa gtggtggcct 5820

aactacggct acactagaag aacagtattt ggtatctgcg ctctgctgaa gccagttacc 5880

ttcggaaaaa gagttggtag ctcttgatcc ggcaaacaaa ccaccgctgg tagcggtggt 5940

ttttttgttt gcaagcagca gattacgcgc agaaaaaaag gatctcaaga agatcctttg 6000

atcttttcta cggggtctga cgctcagtgg aacgaaaact cacgttaagg gattttggtc 6060

atgagattat caaaaaggat cttcacctag atccttttaa attaaaaatg aagttttaaa 6120

tcaatctaaa gtatatatga gtaaacttgg tctgacagtt accaatgctt aatcagtgag 6180

gcacctatct cagcgatctg tctatttcgt tcatccatag ttgcctgact ccccgtcgtg 6240

tagataacta cgatacggga gggcttacca tctggcccca gtgctgcaat gataccgcga 6300

gacccacgct caccggctcc agatttatca gcaataaacc agccagccgg aagggccgag 6360

cgcagaagtg gtcctgcaac tttatccgcc tccatccagt ctattaattg ttgccgggaa 6420

gctagagtaa gtagttcgcc agttaatagt ttgcgcaacg ttgttgccat tgctacaggc 6480

atcgtggtgt cacgctcgtc gtttggtatg gcttcattca gctccggttc ccaacgatca 6540

aggcgagtta catgatcccc catgttgtgc aaaaaagcgg ttagctcctt cggtcctccg 6600

atcgttgtca gaagtaagtt ggccgcagtg ttatcactca tggttatggc agcactgcat 6660

aattctctta ctgtcatgcc atccgtaaga tgcttttctg tgactggtga gtactcaacc 6720

aagtcattct gagaatagtg tatgcggcga ccgagttgct cttgcccggc gtcaatacgg 6780

gataataccg cgccacatag cagaacttta aaagtgctca tcattggaaa acgttcttcg 6840

gggcgaaaac tctcaaggat cttaccgctg ttgagatcca gttcgatgta acccactcgt 6900

gcacccaact gatcttcagc atcttttact ttcaccagcg tttctgggtg agcaaaaaca 6960

ggaaggcaaa atgccgcaaa aaagggaata agggcgacac ggaaatgttg aatactcata 7020

ctcttccttt ttcaatatta ttgaagcatt tatcagggtt attgtctcat gagcggatac 7080

atatttgaat gtatttagaa aaataaacaa ataggggttc cgcgcacatt tccccgaaaa 7140

gtgccacctg acgtctaaga aaccattatt atcatgacat taacctataa aaataggcgt 7200

atcacgaggc cctttcgtc 7219

<210> 8
<211> 15
<212> PRT
<213> Artificial

<220>
<223> 4GS linker

<400> 8

Gly Gly Gly Gly Ser Gly Gly Gly Gly Ser Gly Gly Gly Gly Ser
1               5                   10                  15

<210>  9
<211>  524
<212>  PRT
<213>  Artificial

<220>
<223>  VSG3.G4S (S317A) Protein

<400>  9

Met Gln Ala Ala Ala Leu Leu Leu Leu Val Leu Arg Ala Ile Thr Ser
1               5                   10                  15

Ile Glu Ala Ala Ala Gly Gly Gly Gly Ser Gly Gly Gly Gly Ser Gly
            20                  25                  30

Gly Gly Gly Ser Asp Asp Val Asn Pro Asp Asp Asn Lys Glu Asp Phe
        35                  40                  45

Ala Val Leu Cys Ala Leu Ala Ala Leu Ala Asn Leu Gln Thr Thr Val
    50                  55                  60

Pro Ser Ile Asp Thr Ser Gly Leu Ala Ala Tyr Asp Asn Leu Gln Gln
65                  70                  75                  80

Leu Asn Leu Ser Leu Ser Ser Lys Glu Trp Lys Ser Leu Phe Asn Lys
                85                  90                  95

Ala Ala Asp Ser Asn Gly Ser Pro Lys Gln Pro Pro Glu Gly Phe Gln
            100                 105                 110

Ser Asp Pro Thr Trp Arg Lys Gln Trp Pro Ile Trp Val Thr Ala Ala
        115                 120                 125

Ala Ala Leu Lys Ala Glu Asn Lys Glu Ala Ala Val Leu Ala Arg Ala
    130                 135                 140

Gly Leu Thr Asn Ala Pro Glu Glu Leu Arg Asn Arg Ala Arg Leu Ala
145                 150                 155                 160

Leu Ile Pro Leu Leu Ala Gln Ala Glu Gln Ile Arg Asp Arg Leu Ser
                165                 170                 175

Glu Ile Gln Lys Gln Asn Glu Asp Thr Thr Pro Thr Ala Ile Ala Lys
            180                 185                 190

Ala Leu Asn Lys Ala Val Tyr Gly Gln Asp Lys Glu Thr Gly Ala Val

195                 200                 205

Tyr Asn Ser Ala Asp Cys Phe Ser Gly Asn Val Ala Asp Ser Thr Gln
    210                 215                 220

Asn Ser Cys Lys Ala Gly Asn Gln Ala Ser Lys Ala Thr Thr Val Ala
225                 230                 235                 240

Ala Thr Ile Val Cys Val Cys His Lys Lys Asn Gly Gly Asn Asp Ala
                245                 250                 255

Ala Asn Ala Cys Gly Arg Leu Ile Asn His Gln Ser Asp Ala Gly Ala
            260                 265                 270

Asn Leu Ala Thr Ala Ser Ser Asp Phe Gly Asp Ile Ile Ala Thr Cys
        275                 280                 285

Ala Ala Arg Pro Pro Lys Pro Leu Thr Ala Ala Tyr Leu Asp Ser Ala
    290                 295                 300

Leu Ala Ala Val Ser Ala Arg Ile Arg Phe Lys Asn Gly Asn Gly Tyr
305                 310                 315                 320

Leu Gly Lys Phe Lys Ala Thr Gly Cys Thr Gly Ala Ala Ser Glu Gly
                325                 330                 335

Leu Cys Val Glu Tyr Thr Ala Leu Thr Ala Ala Thr Met Gln Asn Phe
            340                 345                 350

Tyr Lys Ile Pro Trp Val Lys Glu Ile Ser Asn Val Ala Glu Ala Leu
        355                 360                 365

Lys Arg Thr Glu Lys Asp Ala Ala Glu Ser Thr Leu Leu Ser Thr Trp
    370                 375                 380

Leu Lys Ala Ser Glu Asn Gln Gly Asn Ser Val Ala Gln Lys Leu Ile
385                 390                 395                 400

Lys Val Gly Asp Ser Lys Ala Val Pro Pro Ala Gln Arg Gln Thr Gln
                405                 410                 415

Asn Lys Pro Gly Ser Asn Cys Asn Lys Asn Leu Lys Lys Ser Glu Cys
            420                 425                 430

Lys Asp Ser Asp Gly Cys Lys Trp Asn Arg Thr Glu Glu Thr Glu Gly
        435                 440                 445

Asp Phe Cys Lys Pro Lys Glu Thr Gly Thr Glu Asn Pro Ala Ala Gly
    450                 455                 460

Thr Gly Glu Gly Ala Ala Gly Ala Asn Thr Glu Thr Lys Lys Cys Ser
465                 470                 475                 480

Asp Lys Lys Thr Glu Gly Asp Cys Lys Asp Gly Cys Lys Trp Asp Gly
                485                 490                 495

Lys Glu Cys Lys Asp Ser Ser Ile Leu Ala Thr Lys Lys Phe Ala Leu
            500                 505                 510

Thr Val Val Ser Ala Ala Phe Val Ala Leu Leu Phe
        515                 520

<210>  10
<211>  488
<212>  PRT
<213>  Artificial

<220>
<223>  VSG2-1DK Protein

<400>  10

Met Pro Ser Asn Gln Glu Ala Arg Leu Phe Leu Ala Val Leu Val Leu
1               5                   10                  15

Ala Gln Val Leu Pro Ile Leu Val Asp Ser Ala Ala Gly Gly Gly Glu
            20                  25                  30

Asn Leu Tyr Phe Gln Gly Gly Gly Gly Gly Gly Phe Lys Gln Ala Phe
        35                  40                  45

Trp Gln Pro Leu Cys Gln Val Ser Glu Glu Leu Asp Asp Gln Pro Lys
    50                  55                  60

Gly Ala Leu Phe Thr Leu Gln Ala Ala Ala Ser Lys Ile Gln Lys Met
65                  70                  75                  80

Arg Asp Ala Ala Leu Arg Ala Ser Ile Tyr Ala Glu Ile Asn His Gly
                85                  90                  95

Thr Asn Arg Ala Lys Ala Ala Val Ile Val Ala Asn His Tyr Ala Met
            100                 105                 110

Lys Ala Asp Ser Gly Leu Glu Ala Leu Lys Gln Thr Leu Ser Ser Gln
        115                 120                 125

Glu Val Thr Ala Thr Ala Thr Ala Ser Tyr Leu Lys Gly Arg Ile Asp

130                 135                 140

Glu Tyr Leu Asn Leu Leu Leu Gln Thr Lys Glu Ser Gly Thr Ser Gly
145                 150                 155                 160

Cys Met Met Asp Thr Ser Gly Thr Asn Thr Val Thr Lys Ala Gly Gly
                165                 170                 175

Thr Ile Gly Gly Val Pro Cys Lys Leu Gln Leu Ser Pro Ile Gln Pro
            180                 185                 190

Lys Arg Pro Ala Ala Thr Tyr Leu Gly Lys Ala Gly Tyr Val Gly Leu
        195                 200                 205

Thr Arg Gln Ala Asp Ala Ala Asn Asn Phe His Asp Asn Asp Ala Glu
    210                 215                 220

Cys Arg Leu Ala Ser Gly His Asn Thr Asn Gly Leu Gly Lys Ser Gly
225                 230                 235                 240

Gln Leu Ser Ala Ala Val Thr Met Ala Ala Gly Tyr Val Thr Val Ala
                245                 250                 255

Asn Ser Gln Thr Ala Val Thr Val Gln Ala Leu Asp Ala Leu Gln Glu
            260                 265                 270

Ala Ser Gly Ala Ala His Gln Pro Trp Ile Asp Ala Trp Lys Ala Lys
        275                 280                 285

Lys Ala Leu Thr Gly Ala Glu Thr Ala Glu Phe Arg Asn Glu Thr Ala
    290                 295                 300

Gly Ile Ala Gly Lys Thr Gly Val Thr Lys Leu Val Glu Glu Ala Leu
305                 310                 315                 320

Leu Lys Lys Lys Asp Ser Glu Ala Ser Glu Ile Gln Thr Glu Leu Lys
                325                 330                 335

Lys Tyr Phe Ser Gly His Glu Asn Glu Gln Trp Thr Ala Ile Glu Lys
            340                 345                 350

Leu Ile Ser Glu Gln Pro Val Ala Gln Asn Leu Val Gly Asp Asn Gln
        355                 360                 365

Pro Thr Lys Leu Gly Glu Leu Glu Gly Asn Ala Lys Leu Thr Thr Ile
    370                 375                 380

Leu Ala Tyr Tyr Arg Met Glu Thr Ala Gly Lys Phe Glu Val Leu Thr
385                 390                 395                 400

Gln Lys His Lys Pro Ala Glu Ser Gln Gln Gln Ala Ala Glu Thr Glu
                405                 410                 415

Gly Ser Cys Asn Lys Lys Asp Gln Asn Glu Cys Lys Ser Pro Cys Lys
            420                 425                 430

Trp His Asn Asp Ala Glu Asn Lys Lys Cys Thr Leu Asp Lys Glu Glu
        435                 440                 445

Ala Lys Lys Val Ala Asp Glu Thr Ala Lys Asp Gly Lys Thr Gly Asn
    450                 455                 460

Thr Asn Thr Thr Gly Ser Ser Asn Ser Phe Val Ile Ser Lys Thr Pro
465                 470                 475                 480

Leu Trp Leu Ala Val Leu Leu Phe
                485

<210>  11
<211>  529
<212>  PRT
<213>  Artificial

<220>
<223>  ILTat1.24-G4S Protein

<400>  11

Met Val Tyr Arg Asn Ile Leu Gln Leu Ser Val Leu Lys Val Leu Leu
1               5                   10                  15

Ile Val Leu Ile Val Glu Ala Gly Gly Gly Gly Ser Gly Gly Gly Gly
            20                  25                  30

Ser Gly Gly Gly Gly Ser Thr His Phe Gly Val Lys Tyr Glu Leu Trp
        35                  40                  45

Gln Pro Glu Cys Glu Leu Thr Ala Glu Leu Arg Lys Thr Ala Gly Val
    50                  55                  60

Ala Lys Met Lys Val Asn Ser Asp Leu Asn Ser Phe Lys Thr Leu Glu
65                  70                  75                  80

Leu Thr Lys Met Lys Leu Leu Thr Phe Ala Ala Lys Phe Pro Glu Ser
                85                  90                  95

Lys Glu Ala Leu Thr Leu Arg Ala Leu Glu Ala Ala Leu Asn Thr Asp

            100                 105                 110

Leu Arg Ala Leu Arg Asp Asn Ile Ala Asn Gly Ile Asp Arg Ala Val
        115                 120                 125

Arg Ala Thr Ala Tyr Ala Ser Glu Ala Ala Gly Ala Leu Phe Ser Gly
    130                 135                 140

Ile Gln Thr Leu His Asp Ala Thr Asp Gly Thr Thr Tyr Cys Leu Ser
145                 150                 155                 160

Ala Ser Gly Gln Gly Ser Asn Gly Asn Ala Ala Met Ala Ser Gln Gly
                165                 170                 175

Cys Lys Pro Leu Ala Leu Pro Glu Leu Leu Thr Glu Asp Ser Tyr Asn
            180                 185                 190

Thr Asp Val Ile Ser Asp Lys Gly Phe Pro Lys Ile Ser Pro Leu Thr
        195                 200                 205

Asn Ala Gln Gly Gln Gly Lys Ser Gly Glu Cys Gly Leu Phe Gln Ala
    210                 215                 220

Ala Ser Gly Ala Gln Ala Thr Asn Thr Gly Val Gln Phe Ser Gly Gly
225                 230                 235                 240

Ser Arg Ile Asn Leu Gly Leu Gly Ala Ile Val Ala Ser Ala Ala Gln
                245                 250                 255

Gln Pro Thr Arg Pro Asp Leu Ser Asp Phe Ser Gly Thr Ala Arg Asn
            260                 265                 270

Gln Ala Asp Thr Leu Tyr Gly Lys Ala His Ala Ser Ile Thr Glu Leu
        275                 280                 285

Leu Gln Leu Ala Gln Gly Pro Lys Pro Gly Gln Thr Glu Val Glu Thr
    290                 295                 300

Met Lys Leu Leu Ala Gln Lys Thr Ala Ala Leu Asp Ser Ile Lys Phe
305                 310                 315                 320

Gln Leu Ala Ala Ser Thr Gly Lys Lys Thr Ser Asp Tyr Lys Glu Asp
                325                 330                 335

Glu Asn Leu Lys Thr Glu Tyr Phe Gly Lys Thr Glu Ser Asn Ile Glu
            340                 345                 350

Ala Leu Trp Asn Lys Val Lys Glu Glu Lys Val Lys Gly Ala Asp Pro
        355                 360                 365

Glu Asp Pro Ser Lys Glu Ser Lys Ile Ser Asp Leu Asn Thr Glu Glu
    370                 375                 380

Gln Leu Gln Arg Val Leu Asp Tyr Tyr Ala Val Ala Thr Met Leu Lys
385                 390                 395                 400

Leu Ala Lys Gln Ala Glu Asp Ile Ala Lys Leu Glu Thr Glu Ile Ala
                405                 410                 415

Asp Gln Arg Gly Lys Ser Pro Glu Ala Glu Cys Asn Lys Ile Thr Glu
            420                 425                 430

Glu Pro Lys Cys Ser Glu Glu Lys Ile Cys Ser Trp His Lys Glu Val
        435                 440                 445

Lys Ala Gly Glu Lys Asn Cys Gln Phe Asn Ser Thr Lys Ala Ser Lys
    450                 455                 460

Ser Gly Val Pro Val Thr Gln Thr Gln Thr Ala Gly Ala Asp Thr Thr
465                 470                 475                 480

Ala Glu Lys Cys Lys Gly Lys Gly Glu Lys Asp Cys Lys Ser Pro Asp
                485                 490                 495

Cys Lys Trp Glu Gly Gly Thr Cys Lys Asp Ser Ser Ile Leu Ala Asn
            500                 505                 510

Lys Gln Phe Ala Leu Ser Val Ala Ser Ala Ala Phe Val Ala Leu Leu
        515                 520                 525

Phe

<210>  12
<211>  5
<212>  PRT
<213>  Staphylococcus aureus

<220>
<221>  misc_feature
<222>  (3)..(3)
<223>  Xaa can be any naturally occurring amino acid

<400>  12

Leu Pro Xaa Thr Gly
1               5

<210>  13
<211>  5
<212>  PRT
<213>  Staphylococcus aureus

<220>
<221>  misc_feature
<222>  (3)..(3)
<223>  Xaa can be any naturally occurring amino acid

<400>  13

Leu Pro Xaa Thr Ala
1               5

<210>  14
<211>  10
<212>  PRT
<213>  artificial

<220>
<223>  sortagging donor sequence

<400>  14

Gly Gly Gly Ser Leu Pro Ser Thr Gly Gly
1               5                   10

<210>  15
<211>  10
<212>  PRT
<213>  artificial

<220>
<223>  sortagging donor sequence and the sortagging acceptor sequence

<400>  15

Gly Gly Gly Ser Leu Pro Ser Thr Ala Ala
1               5                   10

<210>  16
<211>  7
<212>  PRT
<213>  artificial

<220>
<223>  part of sortase donor sequence

<400>  16

Ser Leu Pro Ser Thr Gly Gly
1               5

<210>  17
<211>  7

<212>  PRT
<213>  artificial

<220>
<223>  sortagging linker sequence

<400>  17

Ser Leu Pro Ser Thr Ala Ala
1               5